(19) Europäisches Patentamt
European Patent Office
Office européen des brevets

(11) Numéro de publication: **0 373 998 B1**

(12) **FASCICULE DE BREVET EUROPEEN**

(45) Date de publication de fascicule du brevet: **11.08.93**

(51) Int. Cl.⁵: **C07C 251/58**, C07D 333/22, C07D 213/53, C07D 307/52, C07D 295/088, A61K 31/15, A61K 31/34, A61K 31/38, A61K 31/40, A61K 31/44, A61K 31/495

(21) Numéro de dépôt: **89403339.8**

(22) Date de dépôt: **01.12.89**

Le dossier contient des informations techniques présentées postérieurement au dépôt de la demande et ne figurant pas dans le présent fascicule.

# (54) Ethers d'oxime de propénone, procédé pour leur préparation et compositions pharmaceutiques les contenant.

(30) Priorité: **02.12.88 FR 8815860**

(43) Date de publication de la demande: **20.06.90 Bulletin 90/25**

(45) Mention de la délivrance du brevet: **11.08.93 Bulletin 93/32**

(84) Etats contractants désignés: **AT BE CH DE ES FR GB GR IT LI LU NL SE**

(56) Documents cités: **EP-A- 0 017 217**

(73) Titulaire: **ELF SANOFI**
**32-34, rue Marbeuf**
**F-75008 Paris(FR)**

(72) Inventeur: **Congy, Christian**
**58, Allée de la Marquise**
**F-34980 Saint Gely du Fesc(FR)**
Inventeur: **Labeeuw, Bernard**
**22, Rue Paul Eluard**
**F-34080 Montpellier(FR)**
Inventeur: **Gueule, Patrick**
**6, Rue des Amandiers**
**F-34820 Teyran(FR)**
Inventeur: **Rinaldi, Murielle**
**2, Rue des Fontardiés**
**F-34680 Saint Georges d'Orques(FR)**

(74) Mandataire: **Gillard, Marie-Louise et al**
**Cabinet Beau de Loménie 158, rue de l'Université**
**F-75340 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 373 998 B1

## Description

La présente invention a pour objet de nouveaux composés éthers d'oximes O-substitués de propène-2 one-1 comportant dans les positions 1 et 3 divers cycles aromatiques et hétéroaromatiques.

Elle concerne également un procédé pour leur préparation et des compositions pharmaceutiques les contenant. En effet, les composés selon l'invention présentent d'intéressantes propriétés thérapeutiques.

En particulier, les composés possèdent une activité sur le système nerveux central et périphérique et sont des antagomistes des récepteurs $5HT_2$.

De nombreux processus biologiques (appétit, sommeil, activité sexuelle, dépression, humeur, hypertension artérielle) sont liés en partie à l'action d'un neurotransmetteur : la sérotonine ou hydroxy-5 tryptamine ou 5HT (R. Glennon, Journal of Medicinal Chemistry, 1987, 30, 1).

Ses effets sont dûs à une interaction du produit avec des sites de liaisons spécifiques (récepteurs 5HT) présents au niveau central et périphérique (tractus gastro-intestinal, poumons, système cardiovasculaire). Actuellement, trois types de sites sont décrits : $5HT_1$, $5HT_2$ et $5HT_3$ avec des sous-types. Les récepteurs de type $5HT_2$ interviendraient dans certains syndrômes cérébraux et ils pourraient jouer un rôle dans l'agrégation plaquettaire (F. DE CLERK et al., Biochemical Pharmacology, 1984 33, 2807), l'hypertension artérielle et les migraines (G. JOHNSON, Reports in Medicinal Chemistry, 1987, 4150) et sur la contraction des muscles lisses (L. COHEN et al., Journal of Pharmacology and Experimental Therapeutics, 1981, 218, 421).

Des dérivés d'éther de diphénylalcanol et de diphénylalcanoneoximeéther possédant des activités antispasmodiques, antiagrégants plaquettaires et des activités sur l'insuffisance cérébrale et sur les démences séniles sont décrits dans le brevet européen 0 017 217.

Plus particulièrement, le composé

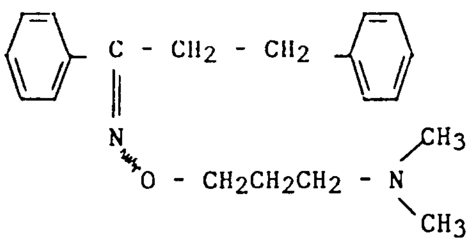

(A)

est décrit parmi les produits possédant des propriétés de vasodilation cérébrale.

On a maintenant trouvé que certains éthers de propénomeoxime sont des composés ayant une haute affinité pour le récepteur $5HT_2$.

On a également trouvé que lesdits éthers de propénoneoxime ont des propriétés pharmacologiques intéressantes notamment une bonne activité d'antiagrégant plaquettaire et sont notamment utiles pour le traitement de toute pathologie 5HT dépendante.

Ainsi, selon un de ses aspects, la présente invention a pour objet des éthers de propénoneoxime répondant à la formule :

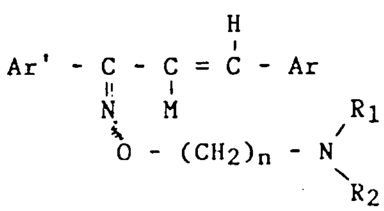

(I)

dans laquelle

- Ar et Ar' peuvent désigner chacun indépendamment soit :

  a) un groupe phényle non substitué, mono ou polysubstitué par un atome d'halogène, un groupement alkyle inférieur (de 1 à 4 atomes de carbone), un groupement nitro, hydroxyle, alcoxy (de 1 à 4 atomes de carbone), acyloxy (de 1 à 4 atomes de carbone), diméthylamino, carboxyalcoxy dans lequel l'alkylène contient de 1 à 4 atomes de carbone ; ou un groupe naphtyle ou l'anthryle-9,

b) un groupe hétéroaromatique choisi parmi les groupes pyridyle, thiényle ou furyle ;

- $R_1$ et $R_2$ désignent chacun indépendamment un atome d'hydrogène, un groupement alkyle inférieur (de 1 à 4 atomes de carbone) ou encore $R_1$ et $R_2$ constituent avec l'atome d'azote auquel ils sont liés, un groupement pyrrolidinyl-1, pipéridino, morpholino ou pipérazinyl-1 ;
- M représente un atome d'hydrogène, un atome de chlore ou de brome, un alkyle inférieur droit ou ramifié de 1 à 6 atomes de carbone ;
- n = 2 ou 3 ;

ainsi que leurs sels avec les acides minéraux ou organiques.

Parmi les groupes hétéroaromatiques, les groupes pyridyl-3, thiényl-2, thiényl-3 ou furyl-2 sont les groupes préférés.

Les acides minéraux ou organiques qui forment les sels d'addition selon la présente invention comprennent aussi bien ceux qui permettent une séparation ou une cristallisation convenable des composés de formule I, tels que l'acide picrique ou l'acide oxalique que ceux qui forment des sels pharmaceutiquement acceptables tels que le chlorhydrate, le bromohydrate, le sulfate, l'hydrogénosulfate, le dihydrogénophosphate, le méthanesulfonate, le méthylsulfate, le maléate, le fumarate, le naphtalènesulfonate, l'iséthionate.

Il est connu que les composés de formule

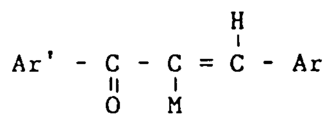

(II)

où Ar et Ar' ont les significations précédemment déterminées et qui sont nommés "chalcones", se trouvent préférentiellement sous la forme trans par rapport à la double liaison du propène (Bull. Soc. Chim. France, 1961, 5, 1369).

Les composés (I) selon l'invention sont des oximes de chalcones et présentent une géométrie trans par rapport à la double liaison carbone-carbone.

Pour ce qui concerne la géométrie de la liaison C=N de l'oxime O-substituée, la représentation

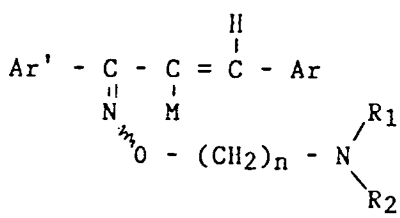

(I)

indique qu'il s'agit d'un mélange en différentes proportions des isomères syn (s) et anti (a) qui sont représentés de la façon suivante (J. Chem. Soc., 1981, 860) :

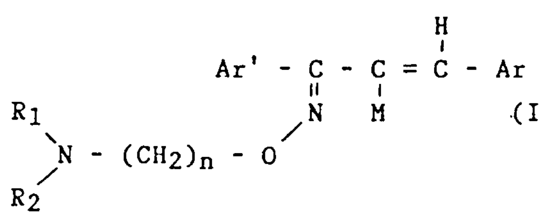

(I)

isomère syn (s)

et

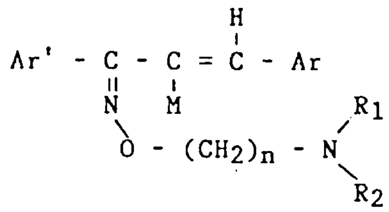 (I)

isomère anti (a)

Selon un mode de réalisation préférentiel, la présente invention a plus particulièrement pour objet un éther d'oxime de propénone selon (I) de formule :

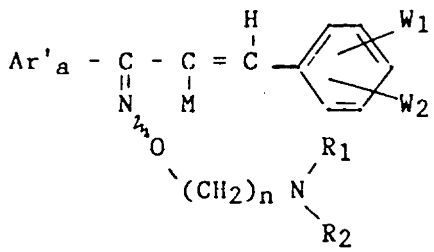 (Ib)

où $Ar'_a$ représente un groupe aromatique choisi parmi le pyridyle, le thiényle, le furyle ou l'anthryle-9 et $W_1$ et $W_2$ représentent chacun indépendamment un atome d'hydrogène, un atome d'halogène, un groupement alkyle inférieur (de 1 à 4 atomes de carbone) un groupe nitro, hydroxyle, alcoxy (de 1 à 4 atomes de carbone), acyloxy (de 1 à 4 atomes de carbone), diméthylamino ou carboxyalcoxy dans lequel l'alkylène contient de 1 à 4 atomes de carbone, ou un de ces sels avec les acides minéraux ou organiques.

Selon un autre mode de réalisation, la présente invention a plus particulièrement pour objet un éther d'oxime de propénone selon (I) de formule :

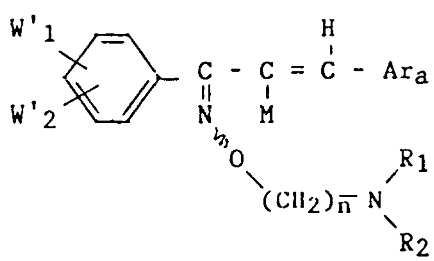 (Ic)

dans laquelle $Ar_a$ représente un groupe choisi parmi le pyridyle, le thiényle, le furyle ou l'anthryle-9 et $W'_1$ et $W'_2$ représentent chacun indépendamment un atome d'hydrogène, un atome d'halogène, un groupement alkyle inférieur (de 1 à 4 atomes de carbone) un groupe nitro, hydroxyle, alcoxy (de 1 à 4 atomes de carbone), acyloxy (de 1 à 4 atomes de carbone) diméthylamino ou carboxyalcoxy dans lequel l'alkylène contient de 1 à 4 atomes de carbone, ou un de ses sels avec les acides minéraux ou organiques.

Des composés préférés, selon l'invention, ont également pour formule:

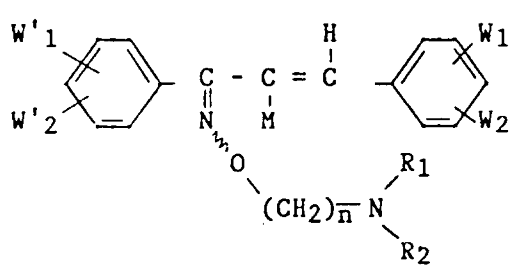 (Id)

dans laquelle $W_1$, $W_2$, $W'_1$, $W'_2$ peuvent désigner indèpendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino, carboxyalcoxy dans lequel l'alkylène contient de 1 à 4 atomes de carbone,

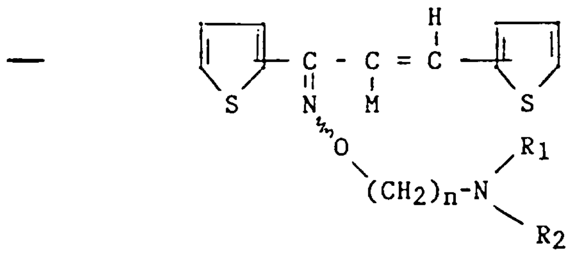

(Ie)

dans laquelle les substituants sont un thiényl-2 ou un thiényl-3,

Il s'agit des composés, sous forme de base ou de sels avec des acides minéraux ou organiques.

Selon un autre de ses aspects, la présente invention a pour objet un procédé pour la préparation des composés de formule (I) et de leurs sels, caractérisé en ce que

a) on traite une chalcone de formule :

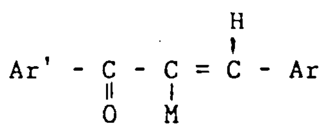

(II)

avec une hydroxylamine de formule :

$H_2NOZ$ (III)

dans laquelle Z représente

- soit une chaîne aminoalkyle de formule :

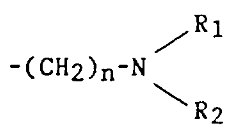

où $R_1$ et $R_2$ ont les significations décrites pour (I)
- soit un atome d'hydrogène
- soit un groupe alkyle substitué de formule :

$-(CH_2)_nX.$

où X représente un groupe partant ; et en ce que

b) on traite ensuite le produit ainsi obtenu de formule :

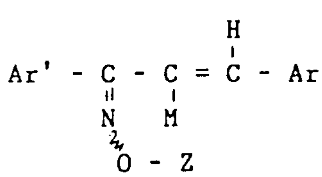

(Ia)

où Ar et Ar' sont tels que définis ci-dessus et où Z représente l'hydrogène ou le groupe $-(CH_2)_nX$, soit, lorsque Z est l'hydrogène, en présence d'un agent de condensation basique, avec une amine de formule

:

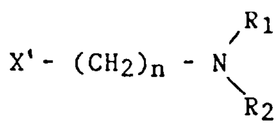 (V)

dans laquelle $R_1$ et $R_2$ sont tels que définis ci-dessus et X'un groupe partant soit, lorsque Z représente un groupe

$- (CH_2)_n - X$

où X est tel que défini ci-dessus, avec une amine de formule :

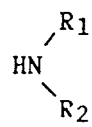

où $R_1$ et $R_2$ sont tels que définis ci-dessus, et en ce que

c) l'on transforme éventuellement le produit ainsi obtenu en a) ou b) en un de ses sels.

Les groupes partant représentés par X et X' peuvent être un des substituants généralement utilisés dans la préparation des alkylamines, par exemple un atome d'halogène ou un groupe hydroxysilylé ou hydroxy estérifié par l'acide méthanesulfonique.

Le schéma réactionnel suivant indique le mode de préparation des composés selon l'invention.

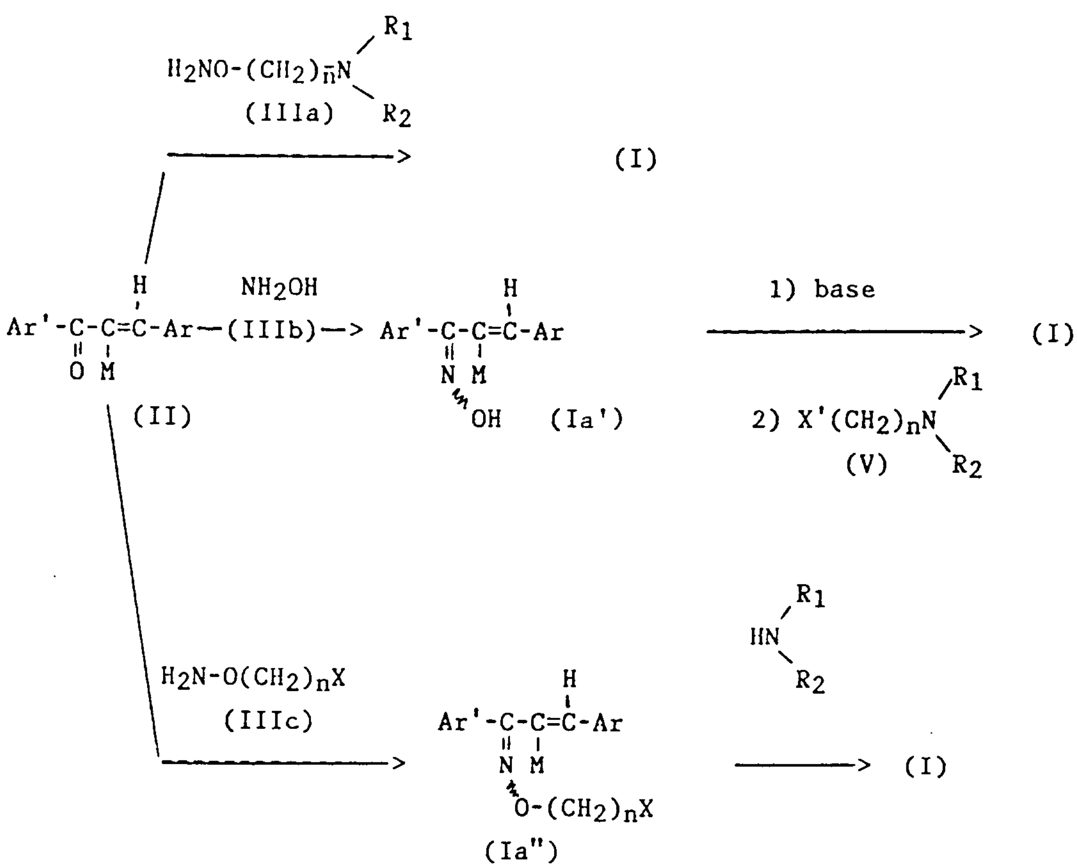

Le choix de la voie de synthèse est fonction de la disponibilité des différentes hydroxylamines ou de leur mode de préparation.

Les sels d'hydroxylamines O-substituées par une chaîne alkylamino de formule :

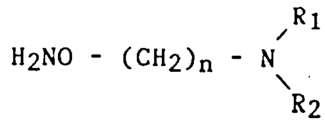

(III a)

peuvent être préparés selon des procédés décrits dans la littérature (Chimia, 1964, fascicule 1 18, 1, 36) et permettent d'obtenir en une seule réaction avec les chalcones (II), dans un solvant tel que l'éthanol chauffé au reflux, les composés (I) objets de l'invention.

La condensation du sel d'hydroxylamine sur une chalcone (II) dans l'alcool ou la pyridine fournit l'oxime de formule :

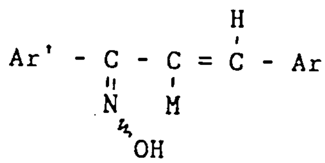

(Ia')

qui est ensuite traitée dans un premier temps par une base comme l'hydrure de sodium ou le carbonate de potassium dans un solvant aprotique polaire tel que le diméthylformamide, le diméthylacétamide ou le diméthylsulfoxide pour être ensuite substituée par une alkylamine (V) comportant un groupement partant X' de formule :

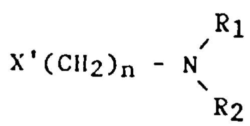

(V)

et conduire aux composés (I) de l'invention.

Une autre variante au procédé général de synthèse met en réaction dans un alcanol, à température ambiante, une chalcone (II) et un sel, par exemple le chlorhydrate d'hydroxylamine O-alkylée de formule :

$HCl, H_2N - O(CH_2)_nX$ (IIIc)

comportant un groupement partant X pour obtenir l'intermédiaire de formule:

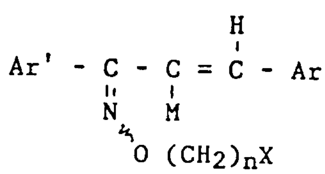

(Ia")

qui est ensuite substitué par une amine soit dans un solvant tel que l'eau ou le diméthylformamide soit en l'absence de solvant et dans ce cas en présence uniquement de l'amine pour conduire finalement aux composés (I) de l'invention.

Le produit de formule (I) ainsi obtenu est isolé, sous forme de base libre ou de sel, selon les techniques conventionnelles.

Lorsque le composé de formule (I) est obtenu sous forme de base libre, la salification est effectuée par traitement avec l'acide choisi dans un solvant organique. Par traitement de la base libre, dissoute par exemple dans un alcool tel que l'isopropanol, avec une solution de l'acide choisi dans le même solvant, on obtient le sel correspondant qui est isolé selon les techniques conventionnelles. Ainsi, on prépare par

exemple le chlorhydrate, le bromhydrate, le sulfate, l'hydrogénosulfate, le dihydrogénophosphate, le méthanesulfonate, le méthylsulfate, l'oxalate, le maléate, le fumarate, le naphtalène-2-sulfonate, l'iséthionate.

A la fin de la réaction du composé (II) avec le composé (III), le composé de formule (I) peut être isolé sous forme d'un de ses sels, par exemple le chlorhydrate ou l'oxalate ; dans ce cas, s'il est nécessaire, la base libre peut être préparée par neutralisation dudit sel avec une base minérale ou organique telle que l'hydroxyde de sodium ou la triéthylamine ou avec un carbonate ou bicarbonate alcalin, tel que le carbonate ou bicarbonate de sodium ou de potassium et éventuellement transformé en l'un de ses autres sels.

La configuration d'un isomère ainsi que les proportions relatives d'un mélange en isomère syn et anti sont déterminées par RMN.

La séparation des isomères syn et anti d'un mélange est effectuée par cristallisation de sels tels que par exemple des oxalates, des maléates, des fumarates ou des chlorhydrates de composés de formule (I).

Les chalcones (II) sont connues ou préparées selon les méthodes décrites dans la littérature (Houben Weyl 10-1, 1181) par condensation de Claisen-Schmidt en faisant réagir un aldéhyde Ar-CHO avec un cétone Ar'-CO-Alk (Alk représente un alkyle de 1 à 7 atomes de carbone).

Aux fins de l'invention, on peut utiliser des dérivés nouveaux de propène-2 one-1 de formule (II). De tels dérivés nouveaux -intermédiaires clés- constituent un autre objet de l'invention, notamment ceux de formule :

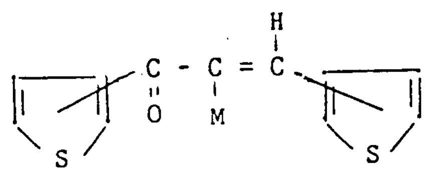

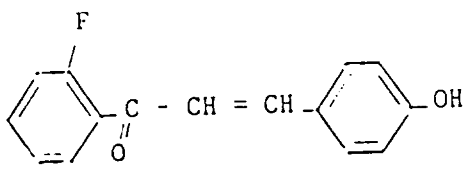

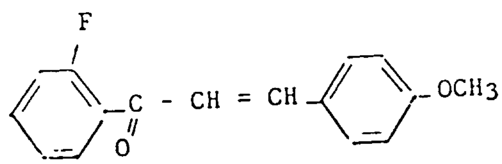

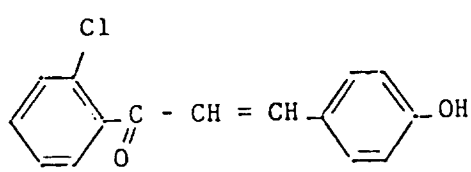

Ces dérivés sont préparés selon des méthodes connues.

Par exemple, celui comportant pour substituant un dérivé thiophénique peut être obtenu en substituant un thiophène-3 carboxaldéhyde avec un acétyl-3 thiophène.

Les composés selon l'invention ont fait l'objet d'essais biochimiques et pharmacologiques et ont été comparés avec le composé (A) de l'art antérieur.

Les composés (I) présentent une bonne activité dans le test de l'antiagrégation plaquettaire réalisé selon T. HALLAM et al. Thrombosis Research, 1982, 27, 435-445, la concentration inhibitrice 50 des composés les plus actifs est de 5 à 50 fois inférieure à celle du composé (A).

Par ailleurs, les composés (I) présentent une haute affinité in vitro et in vivo pour les récepteurs $5HT_2$.

Ces essais sont réalisés selon les conditions expérimentales décrites par J. LEYSEN et al., Molecular Pharmacology, 1982, 21, 301-314 pour ce qui concerne les essais in vitro et selon J. FROST et al., Life Sciences, 1987, 40, 987-997 pour les essais in vivo.

La stimulation du strip d'aorte (= morceau d'aorte) abdominale de lapin met en évidence une activité de 50 à 1500 fois supérieure à celle du produit (A) sur l'antagonisme aux récepteurs 5HT périphériques.Les essais ont été réalisés selon E. APPERLEY et al., Br. J. of Pharmacol., 1976, 58, 211-221.

Les composés selon l'invention sont également antagonistes des récepteurs $5HT_2$ centraux, activité mise en évidence par le test du head-twitch (secousses de la tête) réalisé selon C. GOURET, J. Pharmacol., Paris, 1975, 6, 165-175.

De plus, les composés sont pourvus d'une activité anticonvulsivante mise en évidence par le test de l'antagonisme des crises cloniques induites par le pentétrazole (antagonisme des récepteurs $5HT_2$ centraux) réalisé selon P. WORMS, et al., J. Pharmacol. Exp. Ther., 1982, 220, 660-670.

Les composés de formule (I) sont peu toxiques ; notamment, leur toxicité aiguë est compatible avec leur utilisation comme médicaments, par exemple antiagrégants plaquettaire ou pychotropes.

Pour une telle utilisation, on administre aux mammifères, qui nécessitent ledit traitement, une quantité efficace d'un composé de formule (I) ou d'un de ses sels pharmaceutiquement Acceptables.

Les composés de formule (I) ci-dessus et leurs sels pharmaceutiquement acceptables peuvent être utilisés à des doses journalières de 0,01 à 10 mg par kilo de poids corporel du mammifère à traiter, de préférence à des doses journalières de 0,1 à 5 mg/kg. Chez l'être humain, la dose peut varier de préférence de 0,5 à 500 mg par jour, plus particulièrement de 2,5 à 250 mg selon l'age du sujet à traiter ou le type de traitement : prophylactique ou curatif.

Les composés de formule (I) sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ci-dessus ou un de ses sels pharmaceutiquement acceptables.

Dans les composition pharmaceutiques de la présente invention pour l'administration orale, sublinguale, sous-cutanée, intramusculaire, intraveineuse, transdermique, locale ou rectale, les ingrédients actifs de formule (I) ci-dessus peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Chaque dose unitaire peut contenir de 0,1 à 500 mg d'ingrédient actif, de préférence de 2,5 à 125 mg, en combinaison avec un support pharmaceutique. Cette dose unitaire peut être administrée 1 à 4 fois par jour.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les exemples suivants illustrent l'invention sans toutefois la limiter.

Les spectres RMN sont enregistrés à 250 MHz. Les positions des signaux sont données en millionièmes par rapport au triméthylsilylpropane sulfonate et les spectres sont effectués dans le diméthylsulfoxide deutéré.

Les constantes de couplage J sont données en Hertz (Hz).

Les abréviations suivantes sont utilisées :

s singulet
d doublet
t triplet
m multiplet
se signal élargi

Le symbole "*" dans les tableaux indique que les principaux déplacements chimiques (position des singulets ou du milieu des doublets, triplets ou multiplets) du composé concerné, sont décrits dans le tableau 6.

Les proportions relatives d'isomères syn et anti (% a - % s) ont été déterminés par RMN.

Les points de fusion instantanée (F) des produits recristallisés ont été pris au banc chauffant Kofler et sont exprimés en degrés Celsius.

EXEMPLE 1

trans N,N-diméthylaminoéthoxyimino-1 phényl-1 (hydroxy-4 phényl)-3 propène-2 ; CM 40414 = mélange de 20 % d'isomère syn et de 80 % d'isomère anti.

Ar' = 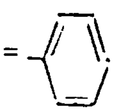 ; Ar = 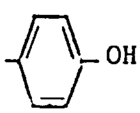 ;

$-NR_1R_2 = -N(CH_3)_2$ ; M = H ; n = 2

a) hydroxy-4 chalcone

Préparée selon Chemistry of Carbon Compounds, E.H. Rodd, 1956, vol. $III^B$, 1186.

b) chlorhydrate de N,N-diméthylamino-2 éthoxyamine.

Préparé selon Bull. Soc. Chim. France, 1958, 5, 664.

c) CM 40414

15 g d'hydroxy-4 chalcone a) et 15 g du composé préparé selon b) sont chauffés à reflux et sous agitation dans 150 ml d'éthanol absolu pendant 5 heures.

Concentrer l'éthanol sous vide, reprendre le résidu dans 200 ml d'acide acétique à 10 % dans l'eau, laver avec du chlorure de méthylène, alcaniser la phase aqueuse avec du bicarbonate de sodium, extraire au chlorure de méthylène, décanter la phase chlorométhylénique$_3$ la laver à l'eau, décanter, sécher sur sulfate de magnésium, filtrer et concentrer sous vide. Recristalliser le résidu de 500 ml d'acétate d'éthyle.

M = 13 g

F = 175 °C.

Le mélange d'isomère contient 20 % de l'isomère syn et 80 % de l'isomère anti.

Spectre de RMN

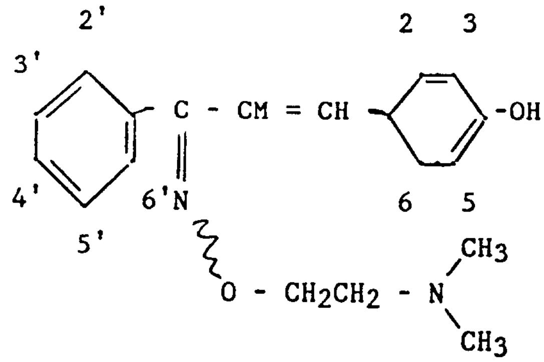

2,05 et 2,15 (6H : 1,2H syn et 4,8H anti, s, $N(CH_3)_2$)
2,4 et 2,55 (2H : 0,4H syn et 1,6H anti, t, J = 6, $CH_2N$)
4,05 et 4,2 (2H : 0,4H syn et 1,6H anti, t, J = 6, $OCH_2$)
6,2 et 6,6 (1H : 0,2H syn et 0,8H anti, d, $J_{trans}$ = 16, H - C =);
6,85 et 7,25 (1H : 0,2H syn et 0,8H anti, d, $J_{trans}$ = 16 H - C);
6,72 (2H, d, $J_{ortho}$ = 8, $H_{3,5}$)
7,30 (2H, d, J : 8, $H_{2,6}$)
7,40 (5H, s, $H_{2',3',4',5',6'}$).

Séparation des isomères syn et anti.

d) hémifumarate de trans N,N-diméthylaminoéthoxyimino-1 phényl-1 (hydroxy-4 phényl)-3 propène-2 ; isomère anti SR 45007 A.

12,3 g de CM 40414 obtenus précédemment sont dissous à chaud dans 220 ml d'isopropanol et 4,6 g d'acide fumarique sont ajoutés. Laisser revenir la solution à température ambiante puis la laisser sous agitation pendant 1H30. Filtrer le fumarate et le rincer à l'éther.

M = 11,6 g
F = 186-187 °C.

Spectre de RMN

2,4 (6H, s, $N(CH_3)_2$)
2,85 (2H, t, J = 6, N-$CH_2$)
4,25 (2H, t, J = 6, $OCH_2$)
6,48 (1H, s, fumarate) ;
6,6 (1H, d, $J_{trans}$ = 16, H - C =) ;
6,7 (2H, d, $J_{ortho}$ = 8, $H_{3,5}$)
7,3 (1H, d, $J_{trans}$ = 16, H - C =) ;
7,35 (2H, d, $J_{ortho}$ = 8, $H_{2,6}$)
7,45 (5H, s, $H_{2',3',4',5',6'}$).

e) hémifumarate de trans N,N-diméthylaminoéthoxyimino-1 phényl-1 (hydroxy-4 phényl)-3 propène-2 isomère syn SR 45008 A.

Concentrer le filtrat du fumarate obtenu précédemment sous vide, reprendre le résidu dans 50 ml d'acétone, séparer un insoluble par filtration puis ajouter de l'éther jusqu'au trouble et laisser cristalliser. Filtrer le précipité et le recristalliser de l'isopropanol.

M = 2,0 g
F = 157-159 °C.

Spectre de RMN

2,2 (6H, s, $N(CH_3)_2$)
2,7 (2H, t, J = 6, $NCH_2$)
4,15 (2H, t, J = 6 $OCH_2$)
6,2 (1H, d, $J_{trans}$ = 16, H - C =) ;

6,45 (1H, s, fumarate) ;
6,7 (2H, d, $J_{ortho}$ = 8, $H_{3,5}$)
6,9 (1H, d, $J_{trans}$ = 16, $\underline{H}$ - C =) ;
7,25 (2H, d, $J_{ortho}$ = 8, $\overline{H}_{2,6}$)
de 7,15 à 7,50 (5H, massif, $H_{2',3',4',5',6'}$).

Isomérisation à partir de l'isomère anti SR 45007 A.

Dans le but de préparer l'isomère syn, obtenu minoritairement au cours de la synthèse, on traite, après l'avoir isolé, l'isomère anti de la façon suivante :

17,7 g de SR 45007 A sont mis en solution dans 200 ml d'éthanol absolu et 9,5 ml d'acide chlorhydrique concentré. Chauffer le mélange réactionnel à reflux pendant 6 heures puis l'abandonner à température ambiante pendant une nuit. Concentrer vous vide, reprendre le résidu dans l'eau, alcaliniser avec du bicarbonate de sodium, filtrer le précipité, le rincer à l'eau et le sécher.

m = 15,1 g d'un mélange de 25 % d'isomère syn et de 75 % d'isomère anti.

Ce mélange est salifié comme précédemment selon d) par l'acide fumarique pour fournir l'isomère anti et le filtrat du fumarate est traité comme précédemment selon e) pour fournir l'isomère syn.

EXEMPLE 2

Oxalate de trans N,N-diméthyl aminoéthoxyimino-1 phényl-1 (méthoxy-4 phényl)-3 propène-2 : SR 45999.

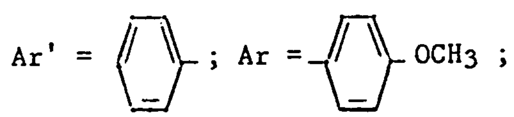

$-NR_1R_2$ = $-N(CH_3)_2$ ;
M = H ; n = 2

Un mélange de 10 g de méthoxy-4 chalcone et de 8,9 g de dichlorhydrate de N,N-diméthylamino-2 éthoxyamine dans 150 ml d'éthanol absolu est chauffé à reflux pendant 7 heures.

Laisser refroidir le mélange réactionnel, filtrer l'excès de réactif et concentrer le filtrat sous vide. Reprendre le résidu dans l'eau, laver à l'éther, alcaliniser la phase aqueuse avec une solution d'ammoniaque concentré, extraire à l'éther, laver à l'eau, sécher sur sulfate de magnésium et concentrer sous vide.

On obtient 12 g d'une huile que l'on chromatographie sur gel de silice afin de séparer les isomères syn et anti.

Eluant : chlorure de méthylène/méthanol 97/3 (v/v).

L'élution du produit le moins polaire permet de recueillir 6,4 g d'une huile à laquelle sont ajoutés 1,7 g d'acide oxalique dans 150 ml d'acétone pour fournir 6,64 g de l'isomère anti : SR 45999 A

F = 162 °C

L'élution du produit le plus polaire permet de recueillir 1,6 g d'une huile à laquelle est ajouté 0,45 g d'acide oxalique dans 20 ml d'acétone pour fournir 1,38 g de l'isomère syn : SR 45996 A.

F = 179 °C.

EXEMPLE 3

Chlorhydrate de trans N,N-diméthylaminoéthoxyimino-1 (méthoxy-4 phényl)-1 (hydroxy-4 phényl)-3 propène-2.

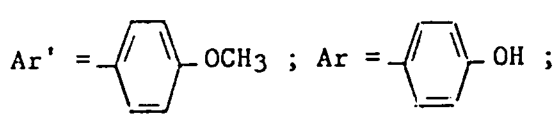

$-NR_1R_2$ = $-N-(CH_3)_2$ ;
M = H ; n = 2

a) Isomère anti ; SR 45175 A

Un mélange de 3 g d'hydroxy-4 méthoxy-4' chalcone et de 3,1 g de dichlorhydrate de N,N-diméthylamino-2 éthoxyamine dans 50 ml d'éthanol est chauffé à reflux pendant 6 heures.

Laisser refroidir le mélange réactionnel, filtrer les cristaux, les agiter dans 20 ml d'eau, filtrer et sécher pour obtenir 2,6 g de l'isomère anti.

F = 216 °C.

b) Mélange de 25 % d'isomère syn et de 75 % d'isomère anti ; SR 45286

Concentrer le filtrat éthanolique obtenu précédemment sous vide, reprendre le résidu dans 100 ml d'eau, extraire deux fois avec de l'acétate d'éthyle, alcaliniser à pH 8 avec du bicarbonate de sodium, décanter la phase aqueuse et l'extraire trois fois avec du chlorure de méthylène, laver à l'eau, décanter, sécher un sulfate de magnésium, concentrer sous vide pour obtenir 0,58 g d'une gomme qui cristallise. Reprendre les cristaux dans 3 ml d'un mélange toluène-éther de pétrole 70-30 (v/v) et filtrer pour obtenir 250 mg d'un mélange de 25 % d'isomère syn et 75 % d'isomère anti.

F = 148 °C.

EXEMPLE 4

Hémifumarate de trans N,N-diméthylaminoéthoxyimino-1 phényl-1 (acétoxy-4 phényl)-3 propène-2. Isomère syn : SR 46024 A.

Ar' = 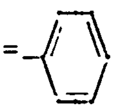 ; Ar = 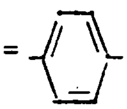 $OCOCH_3$. ;

$-NR_1R_2$ = $-N-(CH_3)_2$ ;

M = H ; n = 2

1,2 g du SR 45008 A précédemment décrit sont agités à température ambiante pendant une nuit dans 12 ml d'anhydride acétique.

Concentrer l'excès d'anhydride acétique sous vide à 20-30 °C, ajouter 30 ml de chlorure de méthylène, laver à l'eau, décanter la phase chlorométhylénique, sécher sur sulfate de magnesium, concentrer le chlorue de méthylène sous vide, reprendre le résidu dans l'éther éthylique, filtrer le précipité et le recristalliser de l'éthanol auquel on ajoute de l'éther jusqu'au trouble m = 0,7 g.

EXEMPLE 5

Oxalate acide de trans N,N-diméthylaminoéthoxyimino-1 di(thiényl-3)-1,3 propène-2 : SR 45557 A.

Ar = Ar' = 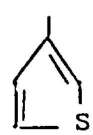 ;

$NR_1R_2$ = $N(CH_3)_2$ ; M = H ; n = 2

a) Préparation du di(thiényl-3)-1,3 propène-2 one-1 trans.

2,25 g de thiophène-3 carboxaldéhyde et 2,52 g d'acétyl-3 thiophène sont mis en solution dans 10 ml d'éthanol absolu.

A la solution refroidie dans la glace on ajoute goutte à goutte une solution de 0,4 g de NaOH dans 1 ml d'eau.

Le mélange réactionnel est agité à 0-5 °C pendant 3 heures. Le précipité est filtré, rincé à l'eau, repris dans l'éther, séché sur sulfate de magnésium. L'éther est concentré sous vide et le résidu est recristallisé du cyclohexane.

M = 2,8 g.

F = 81 °C.

Spectre de RMN

7,58 et 7,83 (6H, m, $H_{thiophène}$, -C H = CH-) ;
8,08 (1H, d, $H_{4'}$)
8,77 (1H, d, $H_4$).

b) SR 45557 A

En condensant selon l'exemple 1 c) précédemment décrit le dérivé thiophénique obtenu selon a) et le dichlorhydrate de N,N-diméthylamino-2 éthoxyamine on obtient le N,N-diméthylaminoéthoxyimino-1 di-(thiényl-3)-1,3 propène-2 trans qui est salifié par l'acide oxalique pour donner l'oxalate acide en mélange de 75 % d'isomère anti et de 25 % d'isomère syn.

F = 138 °C.

EXEMPLE 6

trans N,N-diméthylominoéthoxyimino-1 (thiényl-3)-1 (hydroxy-4 phényl)-3 propène-2 : SR 45047.

Ar' = 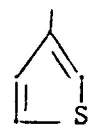 ; Ar = 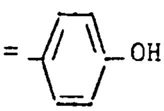 ;

M = H ; n = 2 ; $NR_1R_2$ = $N(CH_3)_2$

a) Préparation du (thiényl-3)-1 (hydroxy-4 phényl)-3 propène-2 one-1 trans.

10 g d'hydroxy-4 benzaldéhyde et 10,4 g d'acétyl-3 thiophène sont mis en solution dans 40 ml d'une solution d'acide chlorhydrique à 4 % dans l'acide acétique.

Agiter le mélange réactionnel à température ambiante pendant 4 jours.

Filtrer le précipité, le rincer avec un mélange acide acétique/eau à 50 % puis le recristalliser dans 30 ml d'éthanol. Filtrer les cristaux.

M = 8,1 g
F = 158 °C.

Spectre de RMN

6,79 (2H, d, $J_{ortho}$ = 8, $H_{3,5}$)
7,6 (4H, m, 2H:HC - C = et $2H_{thiophène}$)
7,67 (2H, d, $J_{ortho}$ = 8, $H_{2,6}$)
8,69 (1H, m, $H_{thiophène}$)
10,05 (1H, se, OH).

b) SR 45047

En condensant selon l'exemple 1 c) précédemment décrit le dérivé thiophénique obtenu selon a) et le chlorhydrate de N,N-diméthylamino-2 éthoxyamine on obtient le SR 45047, mélange de 75 % d'isomère anti et de 25 % d'isomère syn.

F = 170 °C.

EXEMPLE 7

trans N,N-diméthylaminoéthoxyimino-1 (thiényl-2)-1 (hydroxy-4 phényl)-3 propène-2 : SR 45051.

Ar' = 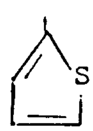 ; Ar = 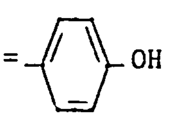 ;

M = H ; n = 2 ; $NR_1R_2$ = $N(CH_3)_2$

a) Préparation du (thiényl-2)-1 (hydroxy-4 phényl)-3 propène-2 one-1 trans.

12,75 g d'acétyl-2 thiophène et 12,10 g d'hydroxy-4 benzaldéhyde sont mis en solution dans 20 ml d'eau. Ajouter une solution de 12,5 g de NaOH dans 12,5 ml d'eau et agiter le mélange réactionnel à température ambiante pendant 4 jours.

Verser le mélange réactionnel dans 300 ml d'acide chlorhydrique à 10%, filtrer le précipité, le dissoudre dans 200 ml de méthanol, ajouter du charbon végétal, filtrer sur célite, concentrer le filtrat sous vide, reprendre le résidu dans l'eau, alcaliniser à pH 11 et extraire à l'éther.

Ajouter de l'acide chlorhydrique jusqu'à apparition d'un précipité et filtrer. Chromatographier sur gel de silice, éluant : hexane-acétate d'éthyle 70-30 (v/v). La fraction contenant le produit attendu est concentrée sous vide et le résidu est recristallisé du chlorure de méthylène.

M = 2,43 g.

Spectre de RMN

6,79 (2H, d, $J_{ortho}$ = 8, $H_{3,5}$)
7,61 (2H s, HC = CH) ;
7,68 (2H, d, $J_{ortho}$ = 8, $H_{2,6}$)
7,24 ; 7,96 ; 8,22 (3H, m, $H_{thiophène}$).

b) SR 45051

En condensant selon l'exemple 1 c) précédemment décrit le dérivé thiophénique obtenu selon a) et le chlorhydrate de N,N- diméthylamino-2 éthoxyamine on obtient le SR 45051, mélange de 20 % d'isomère anti et de 80 % d'isomère syn.

F = 140 °C

EXEMPLE 8

trans N,N-diméthylaminoéthoxyimino-1 phényl-1 (méthoxy-3 hydroxy-4 phényl)-3 propène-2 : SR 45744.

Ar' = 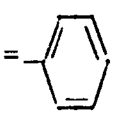 ; Ar = 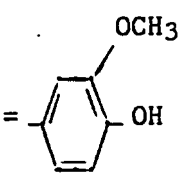 ;

$-NR_1R_2$ = -N $(CH_3)2$ ;
M = H ; n = 2

Ce composé a été préparé selon l'exemple 1. Un mélange de 76 % d'isomère anti et 24 % d'isomère syn est obtenu après recristallisation de l'éthanol.

F = 152 °C.

EXEMPLE 9

Trans N,N-diméthylaminoéthoxyimino-1 (chloro-2 phényl)-1 (hydroxy-4 phényl)-3 propène-2 syn. : SR 46220

Ar' = 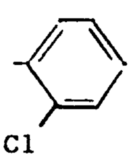 ; Ar = 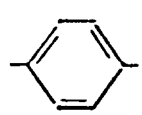 ;

$NR_1R_2$ = $N(CH_3)_2$ ;
M = H ; n = 2

A) Préparation de la chalcone à partir de l'hydroxy-4 benzaldéhyde.

chloro-2' hydroxy-4 chalcone.

20 g de chloro-2 acétophénone et 15,8 g d'hydroxy-4 benzaldéhyde sont mis en solution dans 100 ml d'éthanol saturé en acide chlorhydrique gazeux et le mélange est abandonné pendant 3 jours à température ambiante. L'éthanol est concentré sous vide, le résidu est repris dans 200 ml d'isopropanol puis on ajoute, sous agitation, 500 ml d'eau et filtre le précipité. Après recristallisation de l'isopropanol, on obtient 25,4 g de la chalcone attendue.

F = 141 °C

B) Préparation de la chalcone à partir du méthoxy-4 benzaldéhyde.

a) chloro-2' méthoxy-4 chalcone.

30 g de chloro-2 acétophénone et 26,4 g de méthoxy-4 benzaldéhyde sont introduits dans un mélange, refroidi dans la glace, de 1,8 g de soude en pastille, 88 ml d'eau et 55 ml d'alcool à 95°. La température est maintenue entre 20 et 25 °C, le mélange réactionnel est agité pendant 4 heures, puis abandonné à 5 °C pendant 10 heures. 150 ml d'eau glacée sont alors ajoutés au mélange et un précipité est séparé par filtration puis lavé à l'eau et à l'éthanol pour fournir la chalcone attendue.

m = 50,6 g

F = 83 °C

b) chloro-2' hydroxy-4 chalcone.

30 g de chalcone obtenue précédemment sont mis en solution dans 150 ml de dichlorométhane. La solution est refroidie à - 70 °C puis on ajoute 28,4 ml de tribromure de bore. Après addition, le mélange réactionnel est agité pendant 2 heures à température ambiante, puis versé sur 200 g de glace. Le précipité est filtré puis recristallisé de l'ethanol.

m = 17 g

F = 141 °C

- SR 46220

4 g de chloro-2' hydroxy-4 chalcone obtenue précédemment et 4 g de dichlorhydrate de N,N-diméthylamino-2 éthoxyamine sont mis en solution dans 100 ml d'éthanol et le mélange réactionnel est agité à 40 °C pendant 72 heures.

L'éthanol est concentré sous vide et le résidu est repris dans l'eau et lavé à l'éther. La phase aqueuse est alcalinisée avec une solution de bicarbonate de sodium et extraite au chlorure de méthylène. Après séchage et filtration, la phase organique est concentré sous vide, le rédidu est repris dans l'éther pour fournir 3,25 g de SR 46620 de composition (50% a - 50% s).

EXEMPLE 10

Préparation de l'oxalate du SR 46220 : SR 46220 A.

0,53 g de SR 46620 et 0,138 g d'acide oxalique sont mis en solution dans 5 ml d'acétone. Le mélange est agité à température ambiante pendant 1 heure puis filtré pour fournir 0,45 g d'oxalate qui sont recristallisés dans l'éthanol/éther pour fournir 0,17 g de SR 46220 A (97% syn - 3% anti).

F = 205 °C

EXEMPLE 11

trans N,N-diméthylaminoéthoxyimino-1 (fluoro-2 phényl)-1 (hydroxy-4 phényl)-3 propène-2 syn : SR 46349

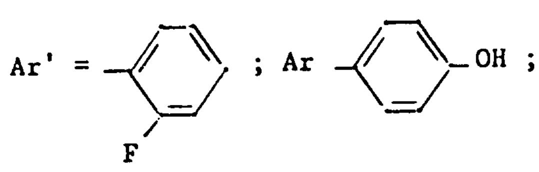

$NR_1R_2 = N(CH_3)_2$ ; M = H ; n = 2

A) Préparation de la chalcone à partir du méthoxy-4 benzaldéhyde.

a) fluoro-2' méthoxy-4 chalcone.

100 g de fluoro-2 acétophénone et 98,55 g de méthoxy-4 benzaldéhyde sont mis en solution dans 360 ml d'éthanol chlorhydrique 2N puis abandonnés pendant 6 jours à 5 °C. On ajoute alors 500 ml d'eau au mélange réactionnel et filtre le précipité pour obtenir 120 g de chalcone attendue.

F = 55 °C

b) fluoro-2' hydroxy-4 chalcone (SR 47035).

En procédant comme précédemment selon l'exemple 9, on obtient par action du tribromure de bore, la chalcone déméthylée.

F = 133 °C (isopropanol).

B) Préparation de la chalcone à partir de l'hydroxy-4 benzaldéhyde. SR 47035

100 g de fluoro-2 acétophénone et 88,4 g d'hydroxy-4 benzaldéhyde sont mis en solution dans l'éthanol chlorhydrique 2N puis abandonnés 9 jours à 5 °C. On additionne alors 1,2 litres d'eau sous agitation, filtre le précipité, le lave par trituration dans l'eau et filtre. Le précipité est séché puis recristallisé dans 2,5 litres de toluène pour fournir 140,6 g de la chalcone attendue.

F = 128 °C

C) SR 46349

85 g de chalcone obtenue précédemment et 85 g de dichlorhydrate de N,N-diméthylamino-2 éthoxyamine sont mis en solution dans 1,5 l d'éthanol chlorhydrique 2N et chauffés à reflux pendant 5 heures. Le mélange est concentré sous vide, le résidu est repris dans l'eau, alcalinisé avec de l'ammoniaque et fractionné de la façon suivante :

pH 5,8 - 6 : 10,5 g anti (SR 46615 exemple n° 29)
pH 6 - 6,5 : 84,9 g 45 % a - 55 % s
pH > 7,5 : 7 g syn. F = 162 °C : SR 46349

Si on alcalinise directement à pH > 8, on obtient la base de composition 45 % syn -55 % anti.

Spectre de RMN du SR 46349

2,00 (6H, s, N $(CH_3)_2$)
2,40 (2H, t, O $\overline{CH_2}$ $CH_2$ N-)
4,05 (2H, t, O $CH_2$ $\overline{CH_2}$ N-)
6,15 (1H, d, H - $\overline{C}$ =)
6,65 (2H, d, $\overline{H}_{3,5}$)
6,90 (1H, d, H - C =)
7,1 à 7,5(6H, m, $H_{3',4',5',6'}$ et $H_{2,6}$)
9,70 (1H, s, ArOH)

L'oximation de la chalcone SR 47035 peut également être réalisée en utilisant le chlorhydrate de N,N-diméthylamino-2 éthoxyamine, dans l'éthanol en présence d'acide méthanesulfonique ou d'acide chlorydrique, pour obtenir l'oxime attendue.

EXEMPLE 12

Hémifumarate de trans N,N- diméthylaminoéthoxyimino-1 (fluoro-2 phényl)-1 (hydroxy-4 phényl)-3 propène-2 syn. SR 46349 B

A) Séparation des isomères syn et anti à partir du SR 46349 (45% syn -55% anti) par formation de l'hémifumarate.

Un mélange homogène de 41,2 g de SR 46349 cristallisé et de 7,23 g d'acide fumarique est préparé. On ajoute alors 300 ml d'éthanol 95° tout en agitant à température ambiante pendant 1 heure 30. Le mélange est alors filtré pour fournir 18 g d'hémifumarate syn qui sont recristallisés de l'éthanol 95°, à 60 °C.

m = 9 g
F = 190 ° C

Spectre de RMN

2,20 (6H, s, N $(CH_3)_2$)
2,68 (2H, t, O-$CH_2$ $CH_2$ N-)
4,20 (2H, t, O-$CH_2$ $CH_2$ N-)
6,25 (1H, d, H - C =)
6,53 (1H, s, fumarate)
6,75 (2H, d, $H_{3,5}$)
6,95 (1H, d, H - C =)
7,2 à 7,6 (6H, m, $H_{3',4',5',6'}$ et $H_{2,6}$)
9,6 à 12 (signal élargi, -$CO_2$ H + DOH)
9,90 (1H, s, Ar-OH)

B) Isomérisation du SR 46615 A (isomère anti de l'hémifumarate du SR 46349).

45 g d'hémifumarate anti du SR 46349 sont mis en solution dans 500 ml d'éthanol 95 ° en présence de 80 ml d'acide chlorhydrique concentré. Le mélange est alors chauffé à reflux pendant 6 heures, à l'abri de la lumière, puis concentré sous vide. Le résidu est repris dans l'eau et lavé à l'éther. La phase aqueuse est alors alcalinisée par de l'ammoniaque et un précipité est séparé par filtration.

On obtient 35,7 g de base 45 % syn -55 % anti qui sont traités comme précédemment pour fournir le SR 46349 B.

EXEMPLE 13

Oxalate de trans N,N-diméthylaminoéthoxyimino-1 (méthoxy-2 phényl)-1 (hydroxy-4 phényl)-3 propène-2 syn : SR 46023 A.

Ar' = 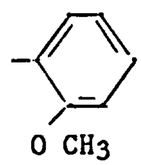 ; Ar = 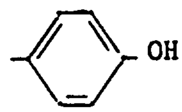 ;

$NR_1R_2$ = N-$(CH_3)_2$ ; M = H ; n = 2

A une suspension de 10 g de N,N- diméthylaminoéthoxyimino-1 (méthoxy-2 phényl)-1 (hydroxy-4 phényl)-3 propène-2 (SR 45743 54 % anti - 46 % syn) dans 200 ml d'acétone, on ajoute 2,6 g d'acide oxalique et agite pendant une heure. On filtre alors l'oxalate qui est agité dans 10 ml de méthanol puis filtré pour obtenir 1,9 g d'oxalate syn.

F = 192 ° C

Spectre de RMN

2,60 (6H, s, N $(CH_3)_2$)
3,25 (2H, t, O-$CH_2$ $CH_2$ N-)
3,70 (3H, s, Ar'-O $CH_3$)
4,30 (2H, t, O-$CH_2$ $CH_2$ N-)
6,20 (1H, d, H - C =)
6,70 (2H, d, $H_{3,5}$)
6,90 (1H, d, H - C =)
6,95 à 7,5 (6H, m, $H_{3',4',5',6'}$ et $H_{2,6}$)
9,80 (1H, s, Ar-OH)
7 à 9,5 (se, H oxalate + DOH)

Les produits selon l'invention, synthétisés dans des conditions expérimentales semblables à celles des exemples 1 à 13 sont répertoriés dans les tableaux 1, 2 et 3 ci-dessous.

Les abréviations suivantes ont été utilisées dans les tableaux pour désigner les solvants de recristallisation :

- EtOH : éthanol
- iPrOH : alcool isopropulique
- DMF : diméthylformamide
- AcOEt : acétate d'éthyle
- $CH_3CN$ : acétonitrile
- tert-BuOH : tertiobutanol
- BuOH : butanol

TABLEAU 1 : Exemples 14 à 55

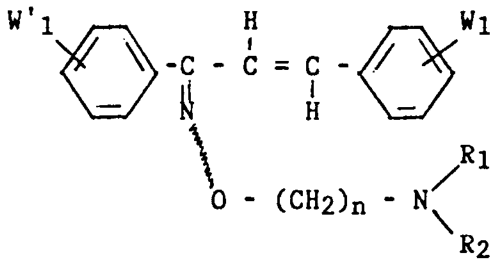

(I)

| Produit n° SR Exemple n° | $W'_1$ | $W_1$ | n | 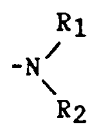 | sel ou base | isomère % a-% s | F,°C solvant recrist. |
|---|---|---|---|---|---|---|---|
| 40258 A 14 | H | H | 2 | $N-(CH_3)_2$ | oxalate acide | a | 170 acétone |
| 45048 A 15 | H | H | 2 | $N-(CH_3)_2$ | hémi oxalate | s | 180 acétone |
| 45560 A 16 | H | H | 3 | $N-(CH_3)_2$ | oxalate acide | 65a-35s | 160 EtOH |
| 45071 17 | H | 2-OH | 2 | $N-(CH_3)_2$ | base | a | 159 AcOEt |
| 40613 18 | H | 3-OH | 2 | $N-(CH_3)_2$ | fumarate | a | 140-2 EtOH |
| 45172 19 | H | 4-OH | 3 | $N-(CH_3)_2$ | base | 92a- 8s | 143 AcOEt |

| | | | | | | | |
|---|---|---|---|---|---|---|---|
| 45287<br>20 | H | 4-OH | 2 | 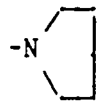 | base | 75a-25s | 181<br>i-PrOH |
| 45288<br>21 | H | 4-OH | 2 | 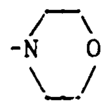 | base | 85a-15s | 126<br>i-PrOH |
| 45289 A<br>22 | H | 4-OH | 3 | 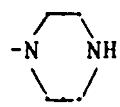 | fumarate | 90a-10s | 218<br>EtOH |
| 46349 A<br>23 | 2-F | 4-OH | 2 | $N-(CH_3)_2$ | oxalate | 40a-60s | * |
| 46349 C<br>24 | 2-F | 4-OH | 2 | $N-(CH_3)_2$ | méthane sulfonate | s | 142<br>tert-BuOH |
| 46349 D<br>25 | 2-F | 4-OH | 2 | $N-(CH_3)_2$ | hémi-sulfate | s | 130-145<br>H2O |
| 46349 E<br>26 | 2-F | 4-OH | 2 | $N-(CH_3)_2$ | phosphate | s | 130-150<br>H2O |
| 46349 F<br>27 | 2-F | 4-OH | 2 | $N-(CH_3)_2$ | maléate acide | s | 140<br>H2O |
| 46349 G<br>28 | 2-F | 4-OH | 2 | $N-(CH_3)_2$ | chlorhy-drate | s | * |
| 46615 A<br>29 | 2-F | 4-OH | 2 | $N-(CH_3)_2$ | hémi-fumarate | a | * |

| | | | | | | | |
|---|---|---|---|---|---|---|---|
| 46564 A 30 | 2-F | 4-OCH3 | 2 | $N-(CH_3)_2$ | oxalate | 20a-80s | * |
| 46220 B 31 | 2-Cl | 4-OH | 2 | $N-(CH_3)_2$ | hémi-fumarate | s | 198-200 EtOH |
| 46251 A 32 | 2-Cl | 4-OCH3 | 2 | $N-(CH_3)_2$ | oxalate | 30a-70s | 118-123 $CH_2Cl_2$/éther |
| 46110 A 33 | 2-Cl | 4-OCH3 | 3 | $N-(CH_3)_2$ | oxalate | 40a-60s | 127 acétone |
| 46190 A 34 | 2-Cl | 4-OCH3 | 3 | $N-(CH_3)_2$ | oxalate | s | 147 BuOH/éther |
| 46278 A 35 | 2-Br | 4-OCH3 | 2 | $N-(CH_3)_2$ | oxalate | 30a-70s | * |
| 46217 A 36 | 2-CH3 | 4-OCH3 | 2 | $N-(CH_3)_2$ | oxalate | 15a-85s | 96 i-PrOH |
| 45743 A 37 | 2-OCH3 | 4-OCH3 | 2 | $N-(CH_3)_2$ | base | 54a-46s | 158 EtOH |
| 46057 38 | 2-OCH3 | 4-OH | 3 | $N-(CH_3)_2$ | base | 80a-20s | 158 EtOH/H2O |
| 46057 A 39 | 2-OCH3 | 4-OH | 3 | $N-(CH_3)_2$ | oxalate | a | 151 EtOH/éther |
| 46109 40 | 2-OCH3 | 4-OH | 3 | $N-(CH_3)_2$ | base | 10a-90s | 129 EtOH |

| | | | | | | | |
|---|---|---|---|---|---|---|---|
| 46289 A<br>41 | 2-<br>$OCH_3$ | 4-OH | 3 | $N-(CH_3)_2$ | base | 52a-48s | * |
| 46219 A<br>42 | 2-<br>$OCH_3$ | 4-<br>$OCH_3$ | 3 | $N-(CH_3)_2$ | oxalate | a | 147<br>$CH_2Cl_2$ |
| 46165 A<br>43 | 2-<br>$OCH_3$ | 4-<br>$OCH_3$ | 2 | $N-(CH_3)_2$ | oxalate | 30a-70s | 146<br>i-PrOH |
| 46175<br>44 | 2-<br>$NO_2$ | 4-OH | 2 | $N-(CH_3)_2$ | base | s | 127-135<br>$CH_2Cl_2$/<br>éther |
| 46400<br>45 | 2-<br>$CF_3$ | 4-OH | 2 | $N-(CH_3)_2$ | base | 50a-50s | * |
| 45678<br>46 | 2-<br>$NO_2$ | 4-OH | 2 | $N-(CH_3)_2$ | base | 93a- 7s | 180<br>$CH_3CN$ |
| 45573<br>47 | 4-F | 4-OH | 2 | $N-(CH_3)_2$ | base | 75a-25s | 188<br>EtOH |
| 45174 A<br>48 | 4-Cl | 4-OH | 2 | $N-(CH_3)_2$ | chlorhy-<br>drate | a | 218<br>EtOH |
| 45574 A<br>49 | 4-I | 4-OH | 2 | $N-(CH_3)_2$ | hémi<br>oxalate | s | 219<br>DMF |
| 45290<br>50 | 4-I | 4-OH | 2 | $N-(CH_3)_2$ | base | a | 183<br>AcOEt |
| 45291<br>51 | 4-OH | 4-OH | | N-(CH3)2 | base | 75a-25s | 265<br>DMF/EtOH |

| | | | | | | | |
|---|---|---|---|---|---|---|---|
| 45681 A 52 | H | 4-N-$(CH_3)_2$ | 2 | N-$(CH_3)_2$ | dichlo-hydrate | 20a-80s | 218 $CH_3CN$/ éther éthylique |
| 45682 A 53 | H | 4-N-$(CH_3)_2$ | 2 | N-$(CH_3)_2$ | oxalate | 70a-30s | 162 $CH_3CN$ |
| 46216 A 54 | H | 4-OCO $H_5C_2$ | 2 | N-$(CH_3)_2$ | oxalate | s | 150 acétone |
| 46025 A 55 | H | 4- $OCH_2$ $HO_2C$ | 2 | N-$(CH_3)_2$ | chlorhy-drate | 60a-40s | - éther |

TABLEAU 2 : Exemples 56 à 63

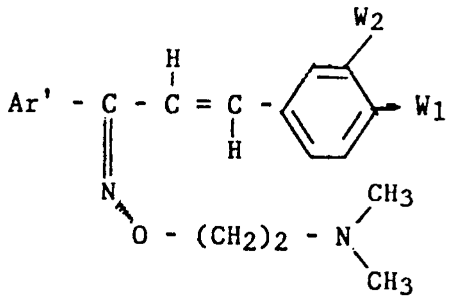

(I)

| Produit n° SR Exemple n° | Ar | W1 | W2 | Sel ou base | isomère % a-% s | F,°C solvant recristal. |
|---|---|---|---|---|---|---|
| 45099 A<br>56 | 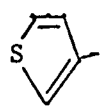 | OH | H | hémi fumarate | anti | 160<br>i-PrOH |
| 45100 A<br>57 | 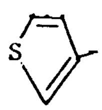 | OH | H | fumarate | syn | 147<br>acétone |
| 45097<br>58 | 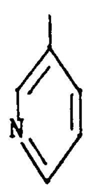 | OH | H | base | 80a-20s | 130<br>$CH_3CN$ |
| 45052<br>59 | 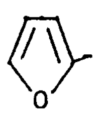 | OH | H | base | 80a-20s | 139<br>AcOEt |

| | | | | | | |
|---|---|---|---|---|---|---|
| 46218 A 60 | 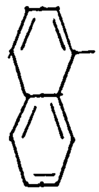 | $OCH_3$ | H | oxalate | 43a-57s | * |
| 46252 A 61 | 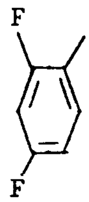 | $OCH_3$ | H | oxalate | s | 156-164 EtOH/éther |
| 46039 62 | 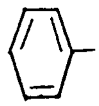 | OH | OH | base | 84a-16s | 180 $CH_3CN$ |
| 46134 63 | 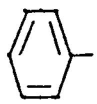 | $OCH_3$ | $OCH_3$ | base | 22a-78s | 55-56 EtOH |

TABLEAU 3 : Exemples 64 à 66

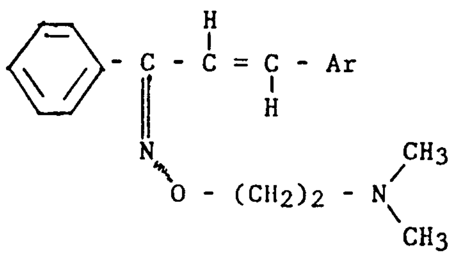

(I)

| Produit n° SR Exemple n° | Ar | sel | isomère % a-% s | F,°C | Solvant recrist. |
|---|---|---|---|---|---|
| 45745 64 | 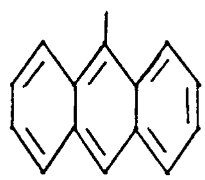 | chlorhydrate | 48a-52s | 178 | i-PrOH |
| 45746 65 | 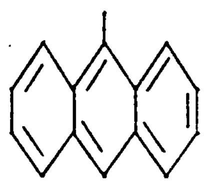 | chlorhydrate | a | 204 | EtOH |
| 45558 A 66 | 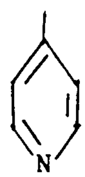 | maléate | 90a-10s | 144 | i-PrOH |

EXEMPLE 67

Oxalate acide de trans (amino-2 éthoxyimino)-1 phényl-1 (hydroxy-4 phényl)-3 propène-2 : SR 45683 A.

Ar' = 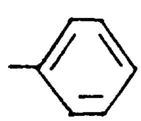 ; Ar = 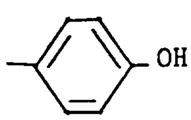 ;

$NR_1R_2 = NH_2$ ; M = H ; n = 2

a) trans (bromo-2 éthoxyimino)-1 phényl-1 (hydroxy-4 phényl)-3 propène-2

20 g d'hydroxy-4 chalcone et 20 g de bromhydrate d'amino-oxy-1 bromo-2 éthane sont mis en solution dans 200 ml d'éthanol absolu. Agiter le mélange réactionnel à température ambiante pendant une nuit,

concentrer l'éthanol sous vide, reprendre le résidu dans l'éther éthylique, filtrer le précipité et le rincer à l'éther éthylique.

M = 33,7 g.

b) SR 45683 A

1 g du produit obtenu précédemment selon a) est mis en solution dans 10 ml d'éthanol saturé d'ammoniac.

La solution est abandonnée 10 jours à température ambiante, l'éthanol est concentré sous vide. Reprendre le résidu dans l'eau, alcaliniser avec du bicarbonate de sodium, extraire à l'acétate d'éthyle, sécher sur sulfate de magnésium, filtrer et concentrer sous vide. Le résidu est dissous à chaud dans 15 ml d'acétone et 250 mg d'acide oxalique sont ajoutés. On laisse revenir la solution à température ambiante et filtre les cristaux qui sont rincés à l'acétone puis recristallisés de l'éthanol pour donner 180 mg de l'isomère anti.

F = 210 ° C.

EXEMPLE 68

Chlorhydrate de trans N,N-diisopropylaminoéthoxyimino-1 phényl-1 (hydroxy-4 phényl)-3 propène-2 : SR 45680 A.

Ar' = 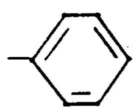 ; Ar = 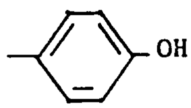 ; $NR_1R_2$ = 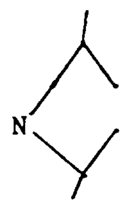 ;

M = H ; n = 2

2 g du produit obtenu précédemment selon l'exemple 67 a) sont mis en solution dans 10 ml de diméthylformamide. Ajouter 10 ml de diisopropylamine et chauffer le mélange réactionnel à 70 ° C pendant 24 heures, concentrer sous vide, reprendre le résidu dans l'eau, sécher sur sulfate de magnésium et concentrer sous vide.

Chromatographier le résidu sur gel de silice, éluant : chlorure de méthylène-méthanol 90-10 (v/v). Les fractions de produit pur sont concentrées sous vide. Le résidu est repris dans l'éther, on ajoute de l'éther chlorhydrique et filtre le chlorhydrate qui est recristallisé de l'acétonitrile pour donner 270 mg de l'isomère anti.

F = 188 ° C.

EXEMPLE 69

Hémifumarate de trans N-méthylaminoéthoxyimino-1 (fluoro-2 phényl)-1 (hydroxy-4 phényl)-3 propène-2. SR 46616 A.

Ar' = 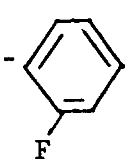 ; Ar = 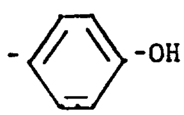 ;

$NR_1R_2$ = $NH-CH_3$ ; M = H ; n = 2

a) Dibromhydrate de bromo-2 N-éthoxycarbonyl N-méthyl éthylamine.

A 540 ml d'eau on ajoute 197 ml de soude 30 % puis 313 g de bromhydrate de bromo-2 N-méthyl éthylamine. On refroidit à 10 ° C et ajoute 155 ml de chloroformiate d'éthyle en maintenant la température en dessous de 15 ° C. Après une nuit d'agitation à température ambiante, on décante la phase aqueuse, extrait à l'éther, lave 3 fois à l eau et sèche sur sulfate de magnésium pour obtenir 142 g du produit attendu.

b) N-(N-éthoxycarbonyl N-méthylamino-2 éthoxy) phtalimide.

A un mélange de 108 g de N-hydroxyphtalimide et 92,5 ml de triéthylamine dans 100 ml de DMF on ajoute le dérivé ci-dessus et chauffe à 87 °C pendant 3 jours. On évapore le DMF, extrait avec du dichlorométhane, lave avec une solution de carbonate de sodium puis à l'eau. On sèche sur sulfate de magnésium et évapore sous vide. On redissout le résidu dans le méthanol et cristallise par addition d'eau pour obtenir 100 g du produit attendu.

c) Bromhydrate de N-méthylamino-2 éthoxyamine.

On chauffe à reflux pendant 1 heure une solution de 100 g du produit précédent dans un mélange de 366 ml HBr 46 % et 246 ml d'acide acétique. On refroidit à 5 °, filtre l'insoluble, évapore le filtrat sous vide. On triture le résidu à chaud dans le tertiobutanol pour obtenir 22,1 g de produit attendu.

d) SR 46616 A.

On chauffe à reflux pendant 1 heure 30 un mélange de 4 g de fluoro-2' hydroxy-4 chalcone et 8,1 g d'hydroxylomine ci-dessus dans 100 ml d'éthanol, puis évapore à sec sous vide. On traite le résidu par de l'eau, extrait 2 fois à l'éther, alcalinise avec du bicarbonate de sodium, extrait au chloroforme. On lave la phase chloroformique par une solution de bicarbonate de sodium, sèche sur $MgSO_4$ et évapore pour obtenir 2,3 g de mélange syn-anti.

Hémifumarate.

On agite pendant 30 mn un mélange de 1,63 g de base ci-dessus et 300 mg d'acide fumarique dans l'éthanol, abandonne une nuit à -15 °C. On filtre 1,45 g d'isomère anti. Par concentration du filtrat on obtient 140 mg de SR 46616A (mélange 70 % syn, 30 % anti).

Les composés répertoriés dans le tableau 4 ont été synthétisés selon les exemples 67, 68 et 69.

TABLEAU 4 : EXEMPLES 70 A 81

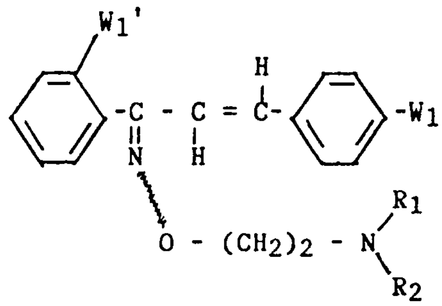

| Produit n° SR Exemple n° | $W_1'$ | $W_1$ | $-N(R_1)(R_2)$ | Sel ou base | isomère % a-% s | F,°C solvant recristal. |
|---|---|---|---|---|---|---|
| 45997 A 70 | H | OH | $NH-CH_3$ | base | 43a-57s | 160 $CH_3CN$ |
| 45998 A 71 | H | OH | $NH-CH_3$ | base | a | 122 éther |
| 46133 72 | H | OH | $NH-CH_3$ | oxalate | 10s-90a | * |
| 46386 A 73 | Cl | OH | $NH_2$ | oxalate | a | * |
| 46385 A 74 | Cl | OH | $NH_2$ | oxalate | 55a-45s | * |
| 46384 A 75 | Cl | OH | $NH-CH_3$ | oxalate | 50a-50s | * |

| | | | | | | |
|---|---|---|---|---|---|---|
| 46387 A<br>76 | $OCH_3$ | OH | $NH_2$ | oxalate | 90a-10s | * |
| 46336 A<br>77 | $OCH_3$ | OH | $NH-CH_3$ | oxalate | 77a-23s | * |
| 46563 A<br>78 | $OCH_3$ | OH | $NH-CH_3$ | hémi-<br>fumarate | 50a-50s | * |
| 46279<br>79 | $OCH_3$ | OH | $N-(C_2H_5)_2$ | base | 80a-20s | * |
| 46132<br>80 | $OCH_3$ | OH | 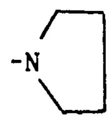 | base | 46a-54s | * |
| 46401<br>81 | $OCH_3$ | OH | H<br>$N-(CH_2)_2-OH$ | base | 73a-27s | * |

EXEMPLE 82

Chlorhydrate de trans N,N-diméthylaminoéthoxyimino-1 diphényl-1,3 propène-2 ; CM 40258.

Ar' = Ar = 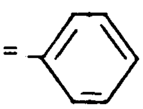 ;

$NR_1R_2 = N(CH_3)2$ ; M = H ; n = 2

Un mélange de 5,1 g d'oxime de la benzalacétophénone, 1,3 g d'hydrure de sodium en suspension dans l'huile (55-60 %) et de 25 ml de diméthylformamide est agité à 20 °C pendant une heure.

Ajouter ensuite à 10 °C 1,2 g d'hydrure de sodium en suspension dans l'huile (55-60 %) puis 4 g de chlorhydrate de diméthylamino-2 chloro-1 éthane et agiter le mélange réactionnel à 20 °C pendant 20 heures.

Verser le mélange réactionnel dans 100 ml d'eau, extraire à l'éther, acidifier avec une solution d'acide chlorhydrique, décanter la phase aqueuse, alcaliniser avec du carbonate de potassium, décanter 6,3 g d'une huile sur laquelle on fait réagir une solution d'acide chlorhydrique dans l'éther éthylique. Recristalliser le chlorhydrate de l'acétone.

M = 4,8 g

F = 209-210 °C.

EXEMPLE 83

Oxalate de trans chloro-2 N,N-diméthylaminoéthoxyimino-1 phényl-1 (méthoxy-4 phényl)-3 propène-2 syn. SR 46356 A.

Ar' = 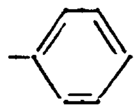 ; Ar = 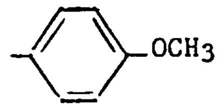 ;

$NR_1R_2$ = N-$(CH_3)_2$ ;
M = C1 ; n = 2

a) Chloro-2 (méthoxy-4 phényl)-3 phényl-1 propène-2 one-1. Préparé selon Z. Chem., 19 Jg., 1979, 3.

b) SR 46356 A.

1,4 g du produit obtenu selon a) et 2 g de dichlorhydrate de N,N-diméthylamino-2 éthoxyamine sont mis en solution dans 40 ml d'éthanol absolu et le mélange réactionnel est chauffé à reflux pendant 24 heures. L'éthanol est concentré sous vide, le résidu est repris dans l'eau, lavé à l'éther, la phase aqueuse est alcalinisée avec du bicarbonate de sodium, extraite au dichlorométhane, séchée et concentrée sous vide. L'huile obtenue est dissoute dans 30 ml d'acétone puis on ajoute 0,5 g d'acide oxalique. L'oxalate est filtré et recristallisé de l'éthanol pour fournir 2,1 g de SR 46356 A.

Les composés répertoriés dans le tableau 5 ont été préparés selon l'exemple 83.

TABLEAU 5 : EXEMPLES 84 à 88

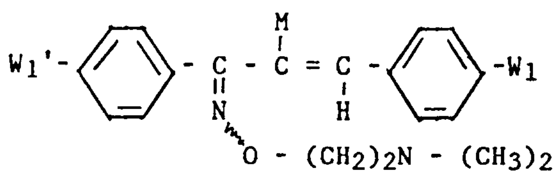

| Produit n°SR Exemple n° | $W_1'$ | $W_1$ | M | sel ou base | isomère %a-%s | F,°C solvant recrist. |
|---|---|---|---|---|---|---|
| 46351 A 84 | H | $OCH_3$ | Cl | oxalate | 80a-20s | * |
| 46348 A 85 | H | $OCH_3$ | Br | oxalate | s | 172-180 EtOH-éther |
| 46254 A 86 | H | OH | $-(CH_2)_2-CH_3$ | oxalate | 80a-20s | 137-147 $CH_2Cl_2$/éther |
| 46253 A 87 | H | OH | $-(CH_2)_2-CH_3$ | oxalate | 30a-70s | 189-193 acétone |
| 46163 A 88 | Cl | OH | $-CH_2-CH_3$ | chlorhydrate | s | 213 EtOH |

TABLEAU 6 : RMN - Déplacements chimiques principaux

| Numéro de l'exemple | $-N-(CH_3)n$ | $-N-CH_2-$ | $-O-CH_2-$ | $-OCH_3$ |
|---|---|---|---|---|
| 23 | 2,20 | 2,68 | 4,20 | - |
| 28 | 2,65 | 3,35 | 4,45 | - |
| 29 | 2,35 | 2,80 | 4,30 | - |
| 30 | 2,60 2,80 | 3,25 3,40 | 4,40 4,50 | 3,75 |
| 35 | 2,60 2,80 | 3,30 3,40 | 4,40 4,50 | 3,75 |
| 41 | 2,10 2,15 | 2,15 2,30 | 4,00 4,10 | 3,70 |
| 45 | 2,00 2,20 | 2,40 2,55 | 4,05 4,20 | - |
| 60 | 2,45 2,85 | 3,20 3,45 | 4,30 4,50 | 3,70 |
| 72 | 2,50 2,55 | 3,20 | 4,20 | - |
| 73 | - | 3,05 | 4,20 | - |
| 74 | - | 2,85 3,05 | 4,10 4,25 | - |
| 75 | 2,50 2,65 | 3,15 3,30 | 4,25 4,35 | - |
| 76 | - | 3,20 | 4,25 | 3,70 |
| 77 | 2,65 | 3,25 | 4,30 | 3,70 |
| 78 | 2,35 2,50 | 2,85 3,10 | 4,10 4,25 | 3,70 |
| 79 | - | 2,40 2,55 | 4,00 4,15 | 3,70 |
| 80 | - | 2,35 2,50 | 4,10 4,20 | 3,70 |
| | | 2,60 2,70 | | |
| 81 | - | 2,50 2,60 | 4,00 4,10 | 3,70 |
| | | 2,70 2,80 | | |
| 84 | 2,65 2,70 | 3,30 3,40 | 4,40 4,50 | 3,80 |

**Revendications**

**Revendications pour les Etats contractants suivants : AT, BE, CH, DE, FR, GB, IT, LI, LU, NL, SE**

**1.** Ethers d'oxime de propènone de géométrie trans par rapport à la double liaison éthylénique et de formule :

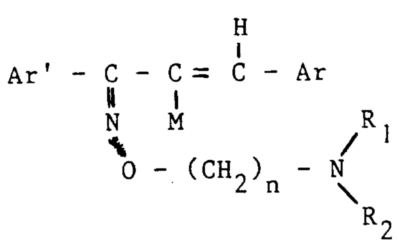

(I)

dans laquelle

- Ar et Ar' peuvent désigner chacun indépendamment soit :
  a) un groupe phényle non substitué, mono ou polysubstitué par un atome d'halogène, un groupement alkyle inférieur (de 1 à 4 atomes de carbone), un groupement nitro, hydroxyle, alcoxy (de 1 à 4 atomes de carbone), acyloxy (de 1 à 4 atomes de carbone), diméthylamino, carboxyalcoxy dans lequel l'alkylène contient de 1 à 4 atomes de carbone ; ou l'anthryle-9 ou un groupe naphtyle ;
  b) un groupe hétéroaromatique choisi parmi les groupes pyridyle, thiényle ou furyle ;
- $R_1$ et $R_2$ désignent chacun indépendamment un atome d'hydrogène, un groupement alkyle inférieur (de 1 à 4 atomes de carbone) ou encore $R_1$ et $R_2$ constituent avec l'atome d'azote auquel ils sont liés, un groupement pyrrolidinyl-1, pipéridino, morpholino ou pipérazinyl-1 ;
- M représente un atome d'hydrogène, un atome de chlore ou de brome, un groupe alkyle inférieur droit ou ramifié de 1 à 6 atomes de carbone ;
- n = 2 ou 3 ;

ainsi que leurs sels avec tes acides minéraux ou organiques.

**2.** Ether d'oxime de propénone selon la revendication 1 de formule :

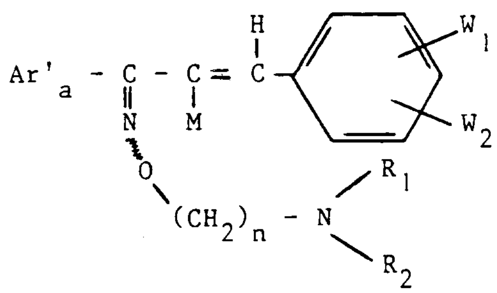

(Ib)

où $Ar'_a$ représente un groupe choisi parmi le pyridyle, le thiényle, le furyle ou l'anthryle-9 et $W_1$ et $W_2$ représentent chacun indépendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino ou carboxyalcoxy dans lequel l'alkylène contient de 1 à 4 atomes de carbone, ou un de ses sels avec les acides minéraux ou organiques.

**3.** Ether d'oxime de propénone suivant la revendication 1, de formule :

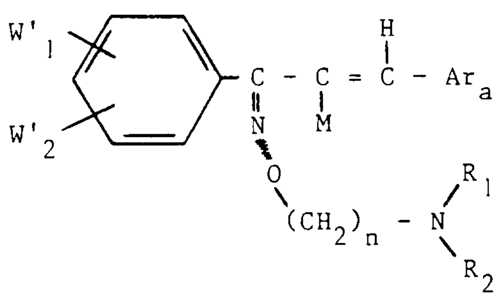

(Ic)

dans laquelle $Ar_a$ représente un groupe choisi parmi le pyridyle, le thiényle, le furyle ou l'anthryle-9 et $W'_1$ et $W'_2$ représentent chacun indépendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino ou carboxyalcoxy dans lequel l'alkylène contient de 1 à 4 atomes de carbone, ou un de ses sels avec les acides minéraux ou organiques.

**4.** Ether d'oxime de propénone selon la revendication 1, de formule :

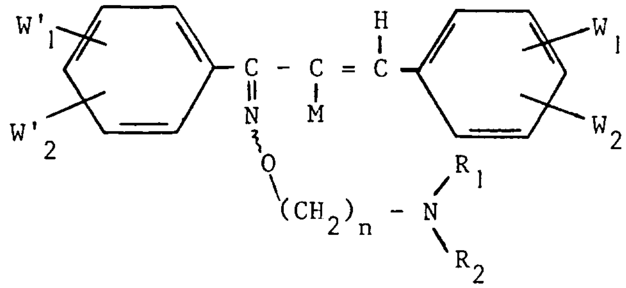

(Id)

dans laquelle $W_1$, $W_2$, $W'_1$, $W'_2$ peuvent désigner chacun indépendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino, carboxyalcoxy dans lequel l'alkylène contient de 1 à 4 atomes de carbone ou un de ses sels avec des acides minéraux ou organiques.

**5.** Ether d'oxime de propénone selon la revendication 1, de formule :

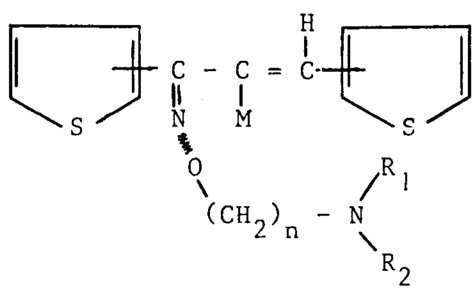

(Ie)

dans laquelle les substituants sont un thiényl-2 ou un thiényl-3, ou un de ses sels avec les acides minéraux ou organiques.

**6.** Trans N,N-diméthylaminoéthoxyimino-1 (fluoro-2 phényl)-1 (hydroxy-4 phényl)-3 propène-2 syn et ses sels avec des acides minéraux ou organiques.

**7.** Méthanesulfonate de trans N,N-diméthylaminoéthoxyimino-1 (fluoro-2 phényl)-1 (hydroxy-4 phényl)-3 propène-2 syn.

**8.** Hémifumarate de trans N,N-diméthylaminoéthoxyimino-1 (fluoro-2 phényl)-1 (hydroxy-4 phényl)-3 propène-2 syn.

**9.** Trans N,N-diméthylaminoéthoxyimino-1 (méthoxy-2 phényl)-1 (hydroxy-4 phényl)-3 propène-2 syn et ses sels avec des acides minéraux ou organiques.

**10.** Trans N,N-diméthylaminoéthoxyimino-1 (chloro-2 phényl)-1 (hydroxy-4 phényl)-3 propène-2 syn et ses sels avec des acides minéraux ou organiques.

**11.** Procédé de préparation des composés selon la revendication 1, caractérisé en ce que :
a) l'on traite une chalcone de formule :

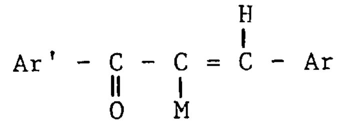

dans laquelle Ar et Ar' sont tels que définis à la revendication 1 avec une hydroxylamine de formule :

$H_2NOZ$ (III)

dans laquelle Z représente

- soit une chaîne aminoalkyle de formule

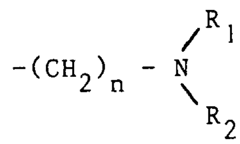

  où $R_1$ et $R_2$ sont tels que définis à la revendication 1
- soit un atome d'hydrogène
- soit un groupe alkyle substitué de formule $-(CH_2)_nX$où n est tel que défini à la revendication 1 et où X représente un groupe partant ;

b) on traite ensuite le produit ainsi obtenu de formule :

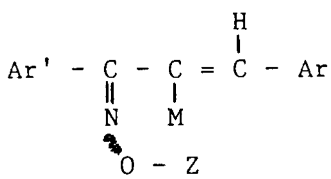

(Ia)

dans laquelle Ar, Ar' sont tels que définis ci-dessus et Z représente l'hydrogène ou un groupe $-(CH_2)_n-X$,

- soit, lorsque Z est l'hydrogène, en présence d'un agent de condensation basique, avec une amine de formule :

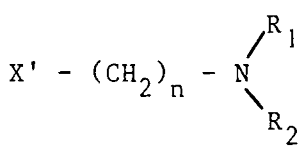

(V)

  dans laquelle $R_1$ et $R_2$ sont tels que définis ci-dessus et X' est un groupe partant ;
- soit lorsque Z représente un groupe $-(CH_2)_n-X$où X est tel que défini ci-dessus, avec une amine de formule

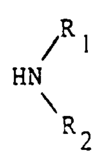

  où $R_1$ et $R_2$ sont tels que définis ci-dessus, et

c) l'on transforme éventuellement le produit ainsi obtenu en a) ou b) en un de ses sels.

**12.** Composition pharmaceutique contenant, en tant que principe actif, un composé selon l'une quelconque des revendications 1 à 10.

**13.** Composition pharmaceutique selon la revendication 12 sous forme d'unité de dosage, dans laquelle le principe actif est mélangé à un excipient pharmaceutique.

**14.** Composition pharmaceutique selon l'une des revendications 12 ou 13, contenant de 0,1 à 500 mg de principe actif par unité de dosage.

**15.** Composition pharmaceutique selon l'une quelconque des revendications 12 à 14, contenant de 2,5 à 125 mg de principe actif par unité de dosage.

**16.** Intermédiaires, utiles à la préparation d'éthers d'oxime de propénone selon la revendication 1 répondant à l'une des formules ci-après :

- 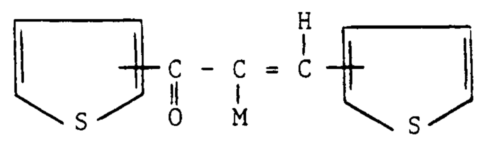

dans laquelle M représente un atome d'hydrogène, un atome de chlore ou de brome ou un groupe alkyle inférieur droit ou ramifié de 1 à 6 atomes de carbone;

- 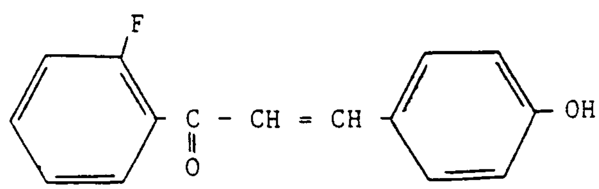
- 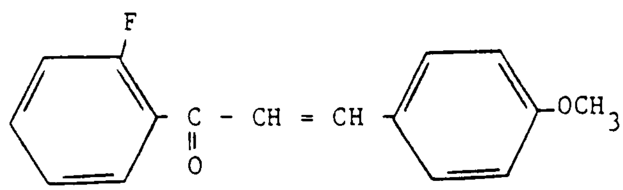
- 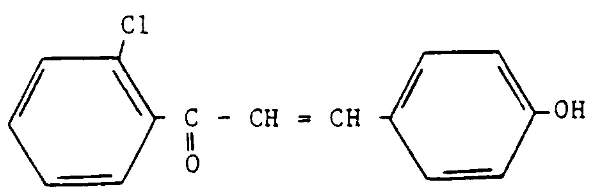 .

## Revendications pour l'Etat contractant suivant : GR

**1.** Ethers d'oxime de propènone de géométrie trans par rapport à la double liaison éthylénique et de formule :

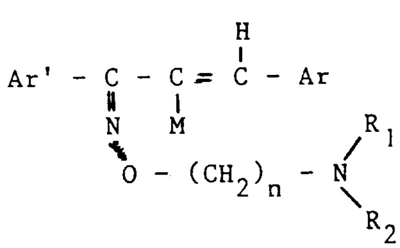 (I)

dans laquelle

- Ar et Ar' peuvent désigner chacun indépendamment soit :

a) un groupe phényle non substitué, mono ou polysubstitué par un atome d'halogène, un groupement alkyle inférieur (de 1 à 4 atomes de carbone), un groupement nitro, hydroxyle,

alcoxy (de 1 à 4 atomes de carbone), acyloxy (de 1 à 4 atomes de carbone), diméthylamino, carboxyalcoxy dans lequel l'alkylène contient de 1 à 4 atomes de carbone ; ou l'anthryle-9 ou un groupe naphtyle ;

b) un groupe hétéroaromatique choisi parmi les groupes pyridyle, thiényle ou furyle ;

- $R_1$ et $R_2$ désignent chacun indépendamment un atome d'hydrogène, un groupement alkyle inférieur (de 1 à 4 atomes de carbone) ou encore $R_1$ et $R_2$ constituent avec l'atome d'azote auquel ils sont liés, un groupement pyrrolidinyl-1, pipéridino, morpholino ou pipérazinyl-1 ;
- M représente un atome d'hydrogène, un atome de chlore ou de brome, un groupe alkyle inférieur droit ou ramifié de 1 à 6 atomes de carbone ;
- n = 2 ou 3 ;

ainsi que leurs sels avec les acides minéraux ou organiques.

**2.** Ether d'oxime de propénone selon la revendication 1 de formule :

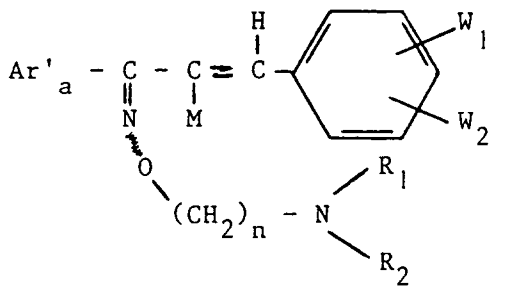

(Ib)

où $Ar'_a$ représente un groupe choisi parmi le pyridyle, le thiényle, le furyle ou l'anthryle-9 et $W_1$ et $W_2$ représentent chacun indépendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino ou carboxyalcoxy dans lequel l'alkylène contient de 1 à 4 atomes de carbone, ou un de ses sels avec les acides minéraux ou organiques.

**3.** Ether d'oxime de propénone suivant la revendication 1, de formule :

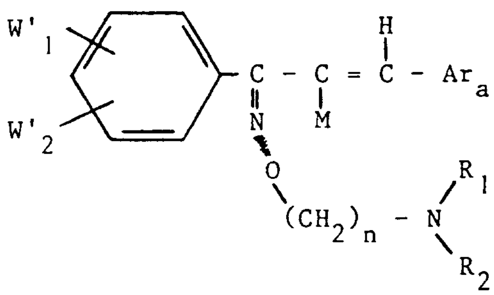

(Ic)

dans laquelle $Ar_a$ représente un groupe choisi parmi le pyridyle, le thiényle, le furyle ou l'anthryle-9 et $W'_1$ et $W'_2$ représentent chacun indépendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino ou carboxyalcoxy dans lequel l'alkylène contient de 1 à 4 atomes de carbone, ou un de ses sels avec les acides minéraux ou organiques.

4. Ether d'oxime de propénone selon la revendication 1, de formule :

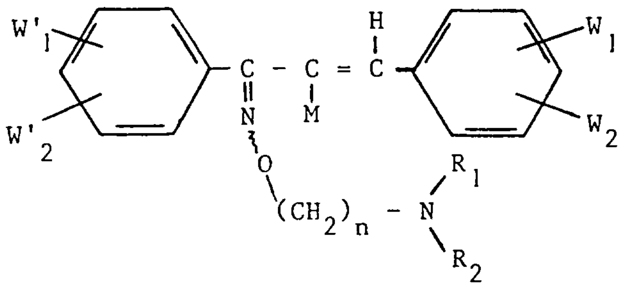

(Id)

dans laquelle $W_1$, $W_2$, $W'_1$, $W'_2$ peuvent désigner chacun indépendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino, carboxyalcoxy dans lequel l'alkylène contient de 1 à 4 atomes de carbone ou un de ses sels avec des acides minéraux ou organiques.

5. Ether d'oxime de propénone selon la revendication 1, de formule :

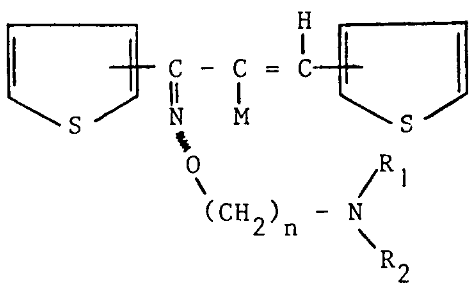

(Ie)

dans laquelle les substituants sont un thiényl-2 ou un thiényl-3, ou un de ses sels avec les acides minéraux ou organiques.

6. Trans N,N-diméthylaminoéthoxyimino-1 (fluoro-2 phényl)-1 (hydroxy-4 phényl)-3 propène-2 syn et ses sels avec des acides minéraux ou organiques.

7. Méthanesulfonate de trans N,N-diméthylaminoéthoxyimino-1 (fluoro-2 phényl)-1 (hydroxy-4 phényl)-3 propène-2 syn.

8. Hémifumarate de trans N,N-diméthylaminoéthoxyimino-1 (fluoro-2 phényl)-1 (hydroxy-4 phényl)-3 propène-2 syn.

9. Trans N,N-diméthylaminoéthoxyimino-1 (méthoxy-2 phényl)-1 (hydroxy-4 phényl)-3 propène-2 syn et ses sels avec des acides minéraux ou organiques.

10. Trans N,N-diméthylaminoéthoxyimino-1 (chloro-2 phényl)-1 (hydroxy-4 phényl)-3 propène-2 syn et ses sels avec des acides minéraux ou organiques.

11. Procédé de préparation des composés selon la revendication 1, caractérisé en ce que :
a) l'on traite une chalcone de formule :

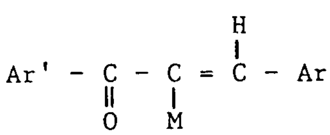

dans laquelle Ar et Ar' sont tels que définis à la revendication 1 avec une hydroxylamine de formule :

$H_2NOZ$ (III)

dans laquelle Z représente
- soit une chaîne aminoalkyle de formule

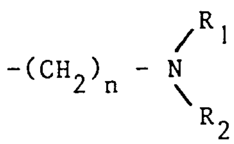

  où $R_1$ et $R_2$ sont tels que définis à la revendication 1
- soit un atome d'hydrogène
- soit un groupe alkyle substitué de formule $-(CH_2)_nX$où n est tel que défini à la revendication 1 et où X représente un groupe partant ;

b) on traite ensuite le produit ainsi obtenu de formule :

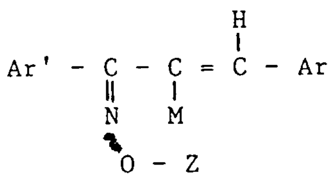

(Ia)

dans laquelle Ar, Ar' sont tels que définis ci-dessus et Z représente l'hydrogène ou un groupe $-(CH_2)_n-X$,
- soit, lorsque Z est l'hydrogène, en présence d'un agent de condensation basique, avec une amine de formule :

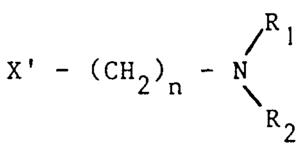

(V)

  dans laquelle $R_1$ et $R_2$ sont tels que définis ci-dessus et X' est un groupe partant ;
- soit lorsque Z représente un groupe $-(CH_2)_n-X$
  où X est tel que défini ci-dessus, avec une amine de formule

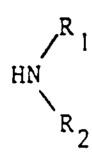

  où $R_1$ et $R_2$ sont tels que définis ci-dessus, et

c) l'on transforme éventuellement le produit ainsi obtenu en a) ou b) en un de ses sels.

**12.** Procédé pour la préparation de compositions pharmaceutiques, caractérisé en ce qu'il consiste à inclure une quantité efficace d'un composé selon l'une quelconque des revendications 1 à à 10, dans un excipient convenable.

**13.** Procédé selon la revendication 12 pour la préparation de compositions pharmaceutiques sous forme d'unité de dosage.

**14.** Procédé selon l'une des revendications 12 ou 13, pour la préparation de compositions pharmaceutiques contenant de 0,1 à 500 mg de principe actif par unité de dosage.

**15.** Procédé selon l'une quelconque des revendications 12 à 14, pour la préparation de compositions pharmaceutiques contenant de 2,5 à 125 mg de principe actif par unité de dosage.

**16.** Intermédiaires, utiles à la préparation d'éthers d'oxime de propénone selon la revendication 1 répondant à l'une des formules ci-après :

- 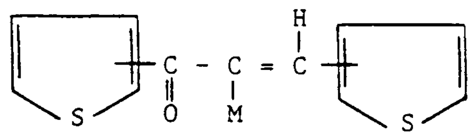

dans laquelle M représente un atome d'hydrogène, un atome de chlore ou de brome ou un groupe alkyle inférieur droit ou ramifié de 1 à 6 atomes de carbone;

- 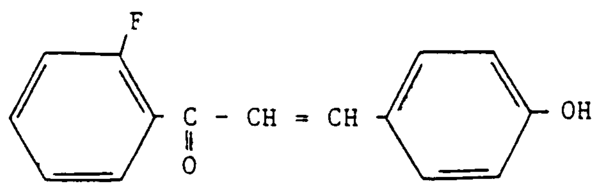
- 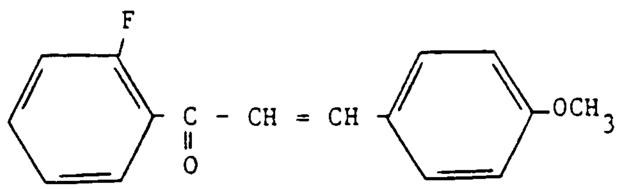
- 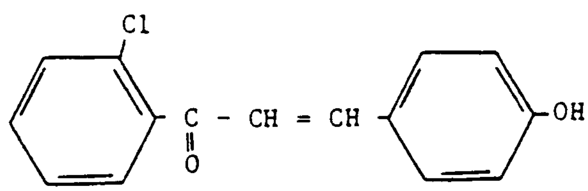 .

## Revendications pour l'Etat contractant suivant : ES

**1.** Procédé de préparation d'éthers d'oxime de propènone de géométrie trans par rapport à la double liaison éthylénique et de formule :

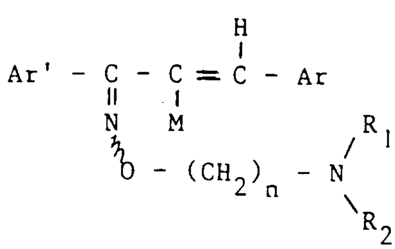 (1)

dans laquelle

- Ar et Ar' peuvent désigner chacun indépendamment soit :

  a) un groupe phényle non substitué, mono ou polysubstitué par un atome d'halogène, un groupement alkyle inférieur (de 1 à 4 atomes de carbone), un groupement nitro, hydroxyle,

alcoxy (de 1 à 4 atomes de carbone), acyloxy (de 1 à 4 atomes de carbone), diméthylamino, carboxyalcoxy dans lequel l'alkylène contient de 1 à 4 atomes de carbone ; ou l'anthryle-9 ou un groupe naphtyle ;

b) un groupe hétéroaromatique choisi parmi les groupes pyridyle, thiényle ou furyle ;

- $R_1$ et $R_2$ désignent chacun indépendamment un atome d'hydrogène, un groupement alkyle inférieur (de 1 à 4 atomes de carbone) ou encore $R_1$ et $R_2$ constituent avec l'atome d'azote auquel ils sont liés, un groupement pyrrolidinyl-1, pipéridino, morpholino ou pipérazinyl-1 ;
- M représente un atome d'hydrogène, un atome de chlore ou de brome, un groupe alkyle inférieur droit ou ramifié de 1 à 6 atomes de carbone ;
- n = 2 ou 3 ;

ainsi que de leurs sels avec les acides minéraux ou organiques, caractérisé en ce que :

a) l'on traite une chalcone de formule :

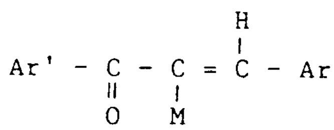

dans laquelle Ar et Ar' sont tels que définis ci-dessus avec une hydroxylamine de formule :

$H_2NOZ$ (III)

dans laquelle Z représente

- soit une chaîne aminoalkyle de formule

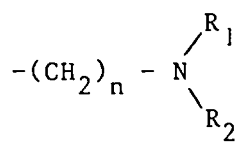

où $R_1$ et $R_2$ sont tels que définis ci-dessus,

- soit un atome d'hydrogène
- soit un groupe alkyle substitué de formule $-(CH_2)_nX$où n est tel que défini ci-dessus et où X représente un groupe partant ;

b) on traite ensuite le produit ainsi obtenu de formule :

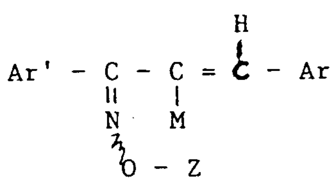

(Ia)

dans laquelle Ar, Ar' sont tels que définis ci-dessus et Z représente l'hydrogène ou un groupe $-(CH_2)_n-X$,

- soit, lorsque Z est l'hydrogène, en présence d'un agent de condensation basique, avec une amine de formule :

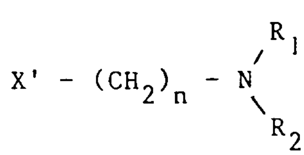

(V)

dans laquelle $R_1$ et $R_2$ sont tels que définis ci-dessus et X' est un groupe partant ;

- soit lorsque Z représente un groupe $-(CH_2)_n-X$
  où X est tel que défini ci-dessus, avec une amine de formule

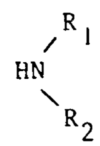

  où $R_1$ et $R_2$ sont tels que définis ci-dessus, et

c) l'on transforme éventuellement le produit ainsi obtenu en a) ou b) en un de ses sels avec des acides minéraux ou organiques.

**2.** Procédé de préparation selon la revendication 1, caractérisé en ce que l'on traite, à l'étape a) une chalcone de formule :

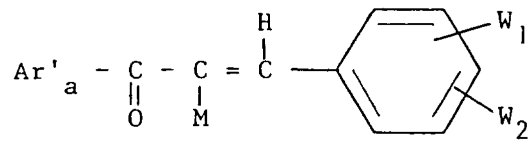

Dans laquelle $Ar'_a$ représente un groupe choisi parmi le pyridyle, le thiényle, le furyle ou l'anthryle-9 et $W_1$ et $W_2$ représentent chacun indépendamment un atome d'hydrogène, un atome d'halogène$_7$ un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino ou carboxyalcoxy dans lequel l'alkylène contient de 1 à 4 atomes de carbone ; M étant tel que défini à la revendication 1.

**3.** Procédé de préparation selon la revendication 1, caractérisé en ce que l'on traite, à l'étape a) une chalcone de formule :

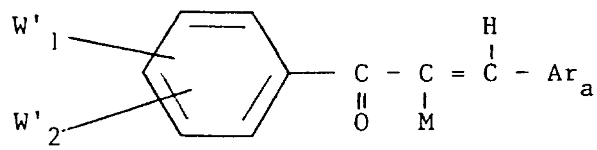

dans laquelle $Ar_a$ représente un groupe choisi parmi le pyridyle, le thiényle, le furyle ou l'anthryle-9 et $W'_1$ et $W'_2$ représentent chacun indépendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino ou carboxyalcoxy dans lequel l'alkylène contient de 1 à 4 atomes de carbone ; M étant tel que défini à la revendication 1.

**4.** Procédé de préparation selon la revendication 1, caractérisé en ce que l'on traite, à l'étape a) une chalcone de formule :

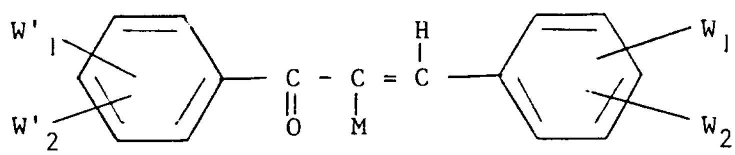

dans laquelle $W_1$, $W_2$, $W'_1$, $W'_2$ peuvent désigner chacun indépendamment un atome d'hydrogène, un atome d'halogène, un groupement alkyle inférieur (de 1 à 4 atomes de carbone), un groupe nitro, hydroxyle, alcoxy (de 1 à 4 atomes de carbone), acyloxy (de 1 à 4 atomes de carbone), diméthylamino, carboxyalcoxy dans lequel l'alkylène contient de 1 à 4 atomes de carbone et M est tel que défini à

la revendication 1.

**5.** Procédé de préparation selon la revendication 1, caractérisé en ce que l'on traite, à l'étape a) une chalcone de formule :

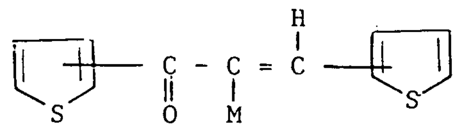

dans laquelle les substituants sont un thiényl-2 ou un thiényl-3, et M est tel que défini à la revendication 1.

**6.** Procédé de préparation selon la revendication 1, caractérisé en ce que :

a) l'on traite une chalcone de formule :

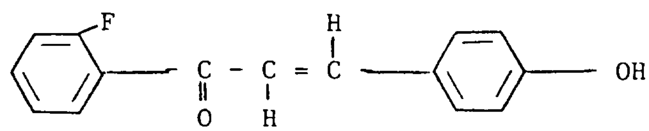

avec une hydroxylamine de formule :

$H_2NOZ$ (III)

dans laquelle Z représente :

- soit une chaîne amino alkyle de formule :

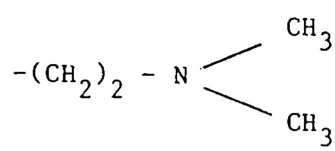

- soit un atome d'hydrogène
- soit un groupe alkyle substitué de formule : $-(CH_2)_2X$ où X représente un groupe partant ;

b) on traite ensuite le produit ainsi obtenu de formule :

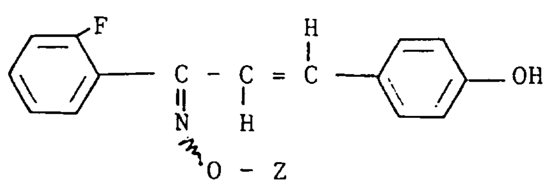 (1a)

dans laquelle Z représente l'hydrogène ou le groupe $-(CH_2)_2X$ ;

- soit, lorsque Z représente l'hydrogène, avec une amine de formule :

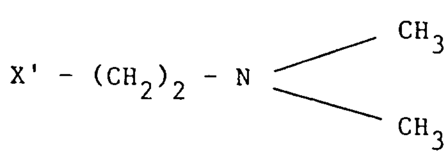

dans laquelle X'
représente un groupe partant ;

- soit, lorsque Z représente un groupe - $(CH_2)_2X$ avec une amine de formule :

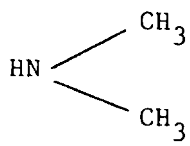

et

c) l'on transforme éventuellement le produit obtenu en a) ou en b) de formule :

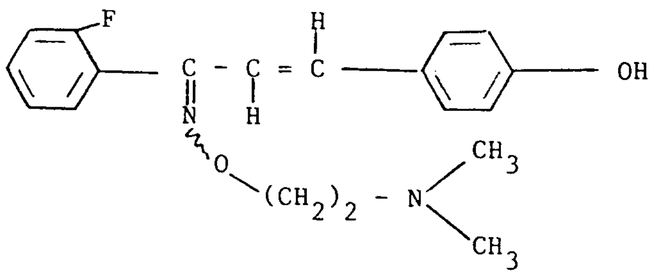

(1)

en l'un de ses sels, tel que le méthanesulfonate ou hémifumarate.

**7.** Procédé de préparation selon la revendication 1, caractérisé en ce que :

a) l'on traite une chalcone de formule :

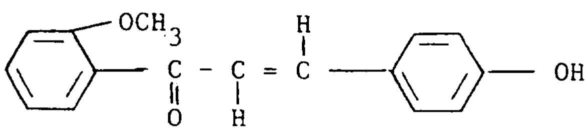

avec une hydroxylamine de formule :

$H_2NOZ$ (III)

dans laquelle Z est tel que défini à la revendication 6.

b) on traite ensuite le produit ainsi obtenu de formule :

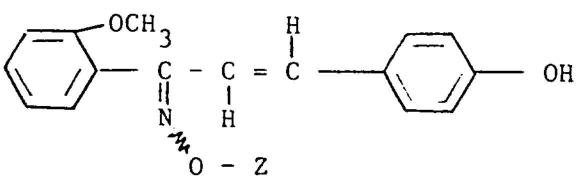

(1a)

dans laquelle Z représente l'hydrogène ou le groupe $(CH_2)_2X$ comme indiqué à la revendication 6 b) ;

c) l'on transforme éventuellement le produit obtenu en a) ou en b) de formule :

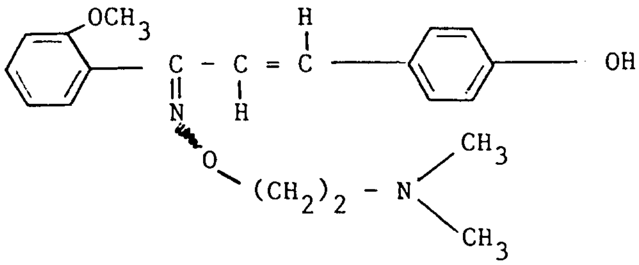

(1)

en l'un de ses sels.

**8.** Procédé de préparation selon la revendication 1, caractérisé en ce que :
a) l'on traite un chalcone de formule :

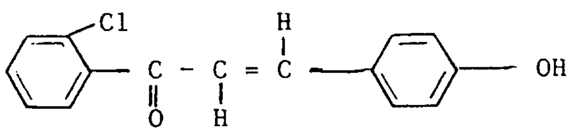

avec une hydroxylamine de formule :
$H_2NOZ$ (III) dans laquelle Z est tel que défini à la revendication 6,
b) on traite ensuite le produit obtenu de formule :

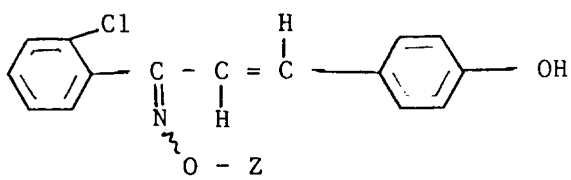

dans laquelle Z représente l'hydrogène ou le groupe - $(CH_2)_2X$ comme indiqué à la revendication 6 b),
c) l'on transforme éventuellement le produit obtenu en a) ou en b) de formule :

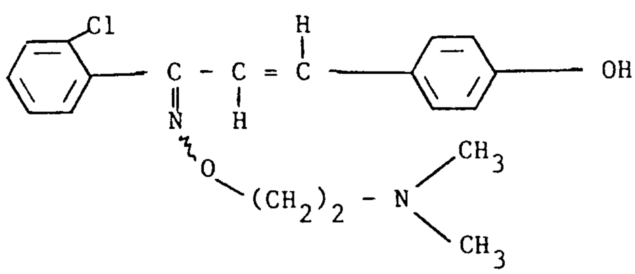

(1)

en l'un de ses sels.

**9.** Procédé pour la préparation d'intermédiaires, utiles à la préparation selon la revendication 1 d'éthers d'oximes de propenone de formule (I) et répondant à l'un de formules ci-après :

— 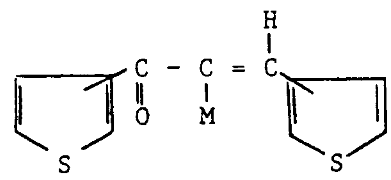

dans laquelle M représente un atome d'hydrogène, un atome de chlore ou de brome ou un groupe alkyle inférieur droit ou ramifié de 1 à 6 atomes de carbone;

— 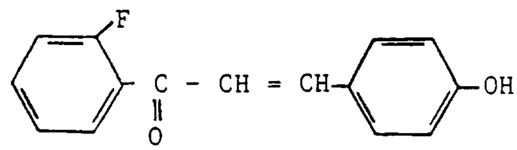

— 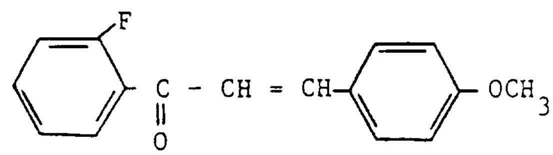

— 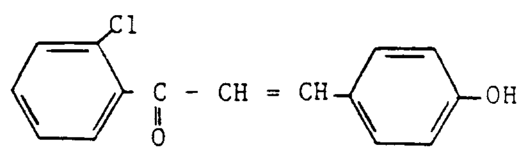

de type :

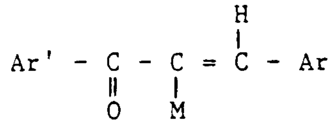

caractérisé en ce qu'il consiste à condenser la cétone correspondante de formule Ar' - CO - Alk dans laquelle Alk représente un groupe alkyle de 1 à 7 atomes de carbone avec l'aldéhyde correspondant de formule Ar - CHO.

**10.** Procédé pour la préparation de compositions pharmaceutiques caractérisé en ce qu'il consiste à inclure une quantité efficace d'un composé de formule (I) ou de l'un de ses sels pharmaceutiquement acceptable, préparé selon l'une quelconque des revendications 1 à 8, dans un excipient convenable.

**11.** Procédé selon la revendication 10, pour la préparation de compositions pharmaceutiques sous forme d'unité de dosage.

**12.** Procédé selon l'une des revendications 10 ou 11, pour la préparation de compositions pharmaceutiques contenant de 0,1 à 500 mg de principe actif par unité de dosage.

**13.** Procédé selon l'une quelconque des revendications 10 à 12, pour la préparation de compositions pharmaceutiques contenant de 2,5 à 125 mg de principe actif par unité de dosage.

## Claims

**Claims for the following Contracting States : AT, BE, CH, DE, FR, GB, IT, LI, LU, NL, SE**

**1.** Propenone oxime ethers having the trans geometry with respect to the double ethylene bond and having the formula:

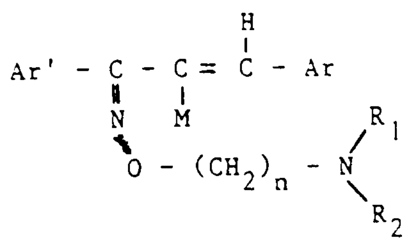

(I)

in which:

- Ar and Ar' can each independently denote either:
  (a) a phenyl group, non-substituted or mono or polysubstituted by a halogen atom, a lower alkyl grouping (containing 1 to 4 carbon atoms), a nitro, hydroxyl, alkoxy (1 to 4 carbon atoms), acyloxy (1 to 4 carbon atoms), dimethylamino grouping or a carboxyalkoxy grouping in which the alkylene contains 1 to 4 carbon atoms; or a 9-anthryl group or a naphthyl group, or
  (b) a heteroaromatic group chosen from the pyridyl, thienyl or furyl groups;
- $R_1$ and $R_2$ each independently denote a hydrogen atom or a lower alkyl grouping (1 to 4 carbon atoms) or $R_1$ and $R_2$ together with the nitrogen atom to which they are bonded constitute a 1-pyrrolidinyl or piperidino or morpholino or 1-piperazinyl grouping;
- M represents a hydrogen atom or a chlorine or bromine atom, or a straight or branched lower alkyl group containing 1 to 6 carbon atoms;
- n = 2 or 3;

and their salts with mineral or organic acids.

**2.** Propenone oxime ether according to claim 1, having the formula:

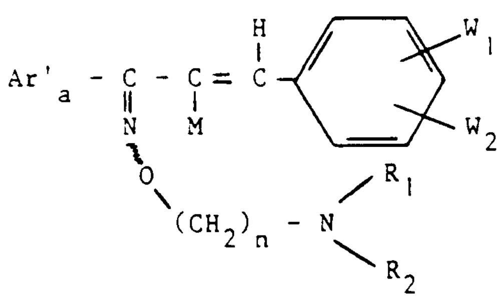

(Ib)

where $Ar'_a$ represents a group chosen from pyridyl, thienyl, furyl or 9-anthryl and $W_1$, and $W_2$ each independently represent a hydrogen atom or a halogen atom or a lower alkyl grouping (1 to 4 carbon atoms) or a nitro or hydroxyl or alkoxy (1 to 4 carbon atoms) or acyloxy (1 to 4 carbon atoms) or dimethylamino grouping or a carboxyalkoxy grouping in which the alkylene contains 1 to 4 carbon atoms, or a salt thereof with mineral or organic acids.

3. Propenone oxime ether according to claim 1, having the formula:

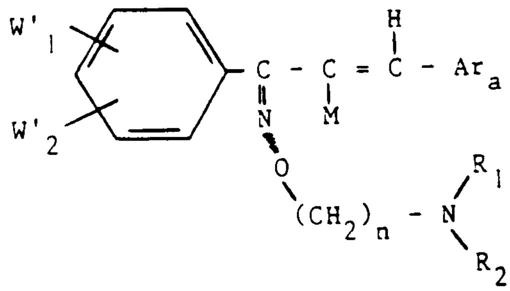

(Ic)

in which $Ar_a$ represents a group chosen from pyridyl, thienyl, furyl or 9-anthryl and $W'_1$ and $W'_2$ each independently represent a hydrogen atom or a halogen atom or a lower alkyl grouping (1 to 4 carbon atoms) or a nitro, hydroxyl, alkoxy (1 to 4 carbon atoms), acyloxy (1 to 4 carbon atoms), dimethylamino grouping or a carboxyalkoxy grouping in which the alkylene contains 1 to 4 carbon atoms, or a salt thereof with mineral or organic acids.

4. Propenone oxime ether according to claim 1, having the formula:

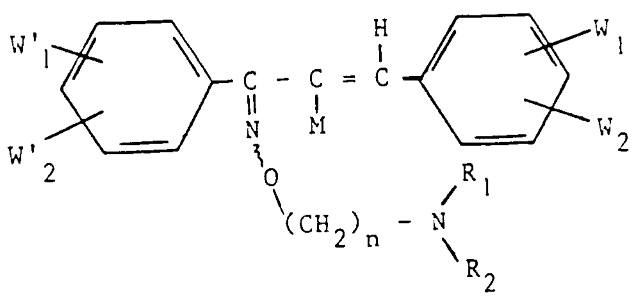

(Id)

in which $W_1$, $W_2$, $W'_1$, $W'_2$ can each independently denote a hydrogen atom, or a halogen atom or a lower alkyl grouping (1 to 4 carbon atoms) or a nitro or hydroxyl or alkoxy (1 to 4 carbon atoms) or acyloxy (1 to 4 carbon atoms) or dimethylamino grouping or a carboxyalkoxy grouping in which the alkylene contains 1 to 4 carbon atoms, or a salt thereof with mineral or organic acids.

5. Propenone oxime ether according to claim 1, having the formula:

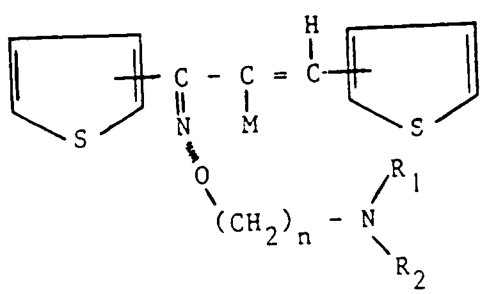

(Ie)

in which the substituents are a 2-thienyl or a 3-thienyl or a salt thereof with mineral or organic acids.

6. Trans 1-N,N-dimethylaminoethoxyimino 1-(2-fluorophenyl) 3-(4-hydroxyphenyl) 2-propene syn and salts thereof with mineral or organic acids.

**7.** Trans 1-N,N-dimethylaminoethoxyimino 1-(2-fluorophenyl) 3-(4-hydroxyphenyl) 2-propane syn methanesulphonate.

**8.** Trans 1-N,N-dimethylaminoethoxyimino 1-(2-fluorophenyl) 3-(4-hydroxyphenyl) 2-propane syn hemifumarate.

**9.** Trans 1-N,N-dimethylaminoethoxyimino 1-(2-methoxyphenyl) 3-(4-hydroxyphenyl) 2-propane syn and salts thereof with mineral or organic acids.

**10.** Trans 1-N,N-dimethylaminoethoxyimino 1-(2-chlorophenyl) 3-(4-hydroxyphenyl) 2-propane syn and salts thereof with mineral or organic acids.

**11.** Method of preparing compounds according to claim 1, characterized in that
a) a chalcone having the formula:

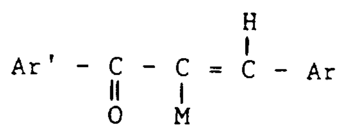

in which Ar and Ar' are as defined in claim 1, is treated with a hydroxylamine having the formula:

$H_2NOZ$ (III)

in which Z represents either
- an aminoalkyl chain having the formula:

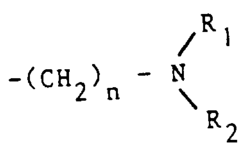

where $R_1$ and $R_2$ are as defined in claim 1 or
- a hydrogen atom or
- a substituted alkyl group having the formula $-(CH_2)_nX$, where n is as defined in claim 1 and where X represents a starting group;

b) the resulting product having the formula:

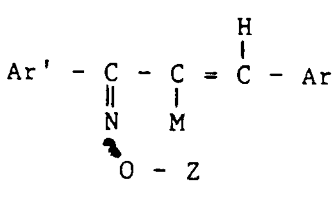

(Ia)

in which Ar, Ar' are as defined hereinbefore and Z represents hydrogen or a $-(CH_2)_nX$ group is then either:
- when Z is hydrogen and in the presence of a basic condensation agent, treated with an amine having the formula:

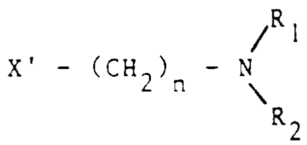 (V)

in which $R_1$ and $R_2$ are as defined hereinbefore and X' is a starting group, or

- when Z represents a group $-(CH_2)_n-X$ where X is as defined hereinbefore, treated with an amine having the formula:

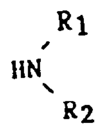

where $R_1$ and $R_2$ are as defined hereinbefore, and

c) the product obtained as in (a) or (b) is converted if required into one of its salts.

**12.** Pharmaceutical composition containing as active principle, a compound according to any of claims 1 to 10.

**13.** Pharmaceutical composition according to claim 12 in the form of a dose unit, in which the active principle is mixed with a pharmaceutical excipient.

**14.** Pharmaceutical composition according to one of claims 12 or 13, containing 0.1 to 500 mg of active principle per unit dose.

**15.** Pharmaceutical composition according to any one of claims 12 to 14, containing 2.5 to 125 mg of active principle per unit dose.

**16.** Intermediates useful in preparing propenone oxime ethers according to claim 1 and having one of the following formulae:

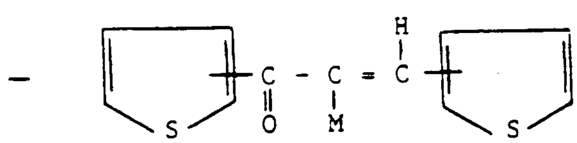

in which M represents a hydrogen atom or a chlorine or bromine atom, or a straight or branched lower alkyl group containing 1 to 6 carbon atoms;

- 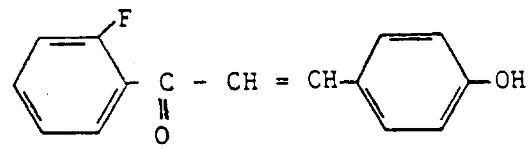
- 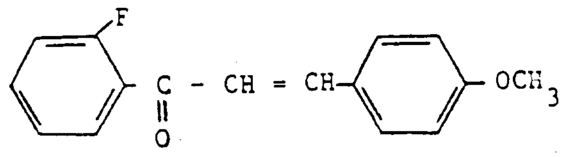
- 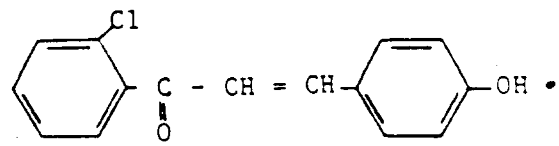

**Claims for the following Contracting State : GR**

**1.** Propenone oxime ethers having the trans geometry with respect to the double ethylene bond and having the formula:

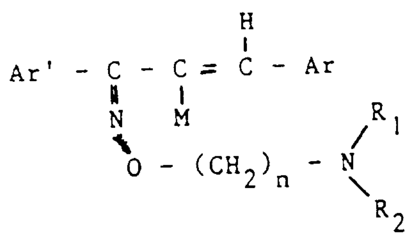

(I)

in which:

- Ar and Ar' can each independently denote either:
  (a) a phenyl group, non-substituted or mono or polysubstituted by a halogen atom, a lower alkyl grouping (containing 1 to 4 carbon atoms), a nitro, hydroxyl, alkoxy (1 to 4 carbon atoms), acyloxy (1 to 4 carbon atoms), dimethylamino grouping or a carboxyalkoxy grouping in which the alkylene contains 1 to 4 carbon atoms; or a 9-anthryl group or a naphthyl group, or
  (b) a heteroaromatic group chosen from the pyridyl, thienyl or furyl groups;
- $R_1$ and $R_2$ each independently denote a hydrogen atom or a lower alkyl grouping (1 to 4 carbon atoms) or $R_1$ and $R_2$ together with the nitrogen atom to which they are bonded constitute a 1-pyrrolidinyl or piperidino or morpholino or 1-piperazinyl grouping;
- M represents a hydrogen atom or a chlorine or bromine atom, or a straight or branched lower alkyl group containing 1 to 6 carbon atoms;
- n = 2 or 3;

and their salts with mineral or organic acids.

**2.** Propenone oxime ether according to claim 1, having the formula:

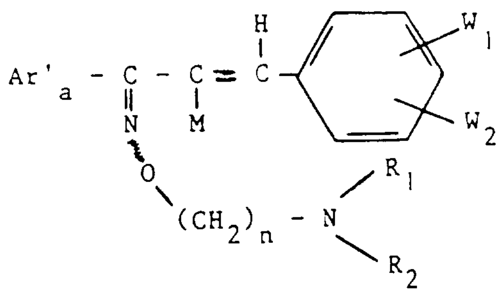

(Ib)

where $Ar'_a$ represents a group chosen from pyridyl, thienyl, furyl or 9-anthryl and $W_1$, and $W_2$ each independently represent a hydrogen atom or a halogen atom or a lower alkyl grouping (1 to 4 carbon atoms) or a nitro or hydroxyl or alkoxy (1 to 4 carbon atoms) or acyloxy (1 to 4 carbon atoms) or dimethylamino grouping or a carboxyalkoxy grouping in which the alkylene contains 1 to 4 carbon atoms, or a salt thereof with mineral or organic acids.

**3.** Propenone oxime ether according to claim 1, having the formula:

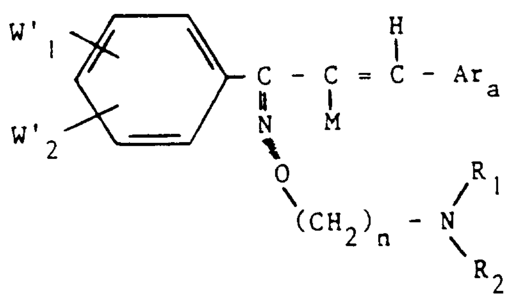

(Ic)

in which $Ar_a$ represents a group chosen from pyridyl, thienyl, furyl or 9-anthryl and $W'_1$ and $W'_2$ each independently represent a hydrogen atom or a halogen atom or a lower alkyl grouping (1 to 4 carbon atoms) or a nitro, hydroxyl, alkoxy (1 to 4 carbon atoms), acyloxy (1 to 4 carbon atoms), dimethylamino grouping or a carboxyalkoxy grouping in which the alkylene contains 1 to 4 carbon atoms, or a salt thereof with mineral or organic acids.

**4.** Propenone oxime ether according to claim 1, having the formula:

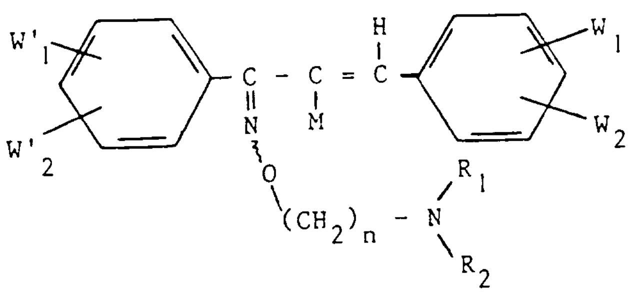

(Id)

in which $W_1$, $W_2$, $W'_1$ $W'_2$ can each independently denote a hydrogen atom, or a halogen atom or a lower alkyl grouping (1 to 4 carbon atoms) or a nitro or hydroxyl or alkoxy (1 to 4 carbon atoms) or acyloxy (1 to 4 carbon atoms) or dimethylamino grouping or a carboxyalkoxy grouping in which the alkylene contains 1 to 4 carbon atoms, or a salt thereof with mineral or organic acids.

**5.** Propenone oxime ether according to claim 1, having the formula:

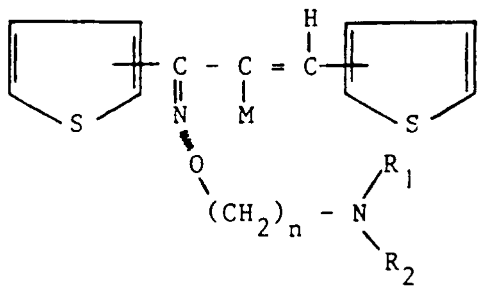

(Ie)

in which the substituents are a 2-thienyl or a 3-thienyl or a salt thereof with mineral or organic acids.

**6.** Trans 1-N,N-dimethylaminoethoxyimino 1-(2-fluorophenyl) 3-(4-hydroxyphenyl) 2-propene syn and salts thereof with mineral or organic acids.

**7.** Trans 1-N,N-dimethylaminoethoxyimino 1-(2-fluorophenyl) 3-(4-hydroxyphenyl) 2-propene syn methanesulphonate.

**8.** Trans 1-N,N-dimethylaminoethoxyimino 1-(2-fluorophenyl) 3-(4-hydroxyphenyl) 2-propene syn hemifumarate.

**9.** Trans 1-N,N-dimethylaminoethoxyimino 1-(2-methoxyphenyl) 3-(4-hydroxyphenyl) 2-propene syn and salts thereof with mineral or organic acids.

**10.** Trans 1-N,N-dimethylaminoethoxyimino 1-(2-chlorophenyl) 3-(4-hydroxyphenyl) 2-propene syn and salts thereof with mineral or organic acids.

**11.** Method of preparing compounds according to claim 1, characterized in that
a) a chalcone having the formula:

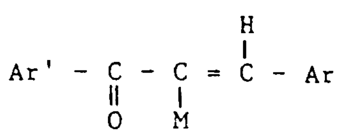

in which Ar and Ar' are as defined in claim 1, is treated with a hydroxylamine having the formula:

$H_2NOZ$ (III)

in which Z represents either

- an aminoalkyl chain having the formula:

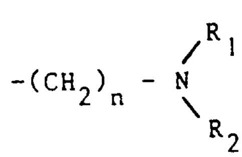

  where $R_1$ and $R_2$ are as defined in claim 1 or
- a hydrogen atom or
- a substituted alkyl group having the formula $-(CH_2)_nX$, where n is as defined in claim 1 and where X represents a starting group;

b) the resulting product having the formula:

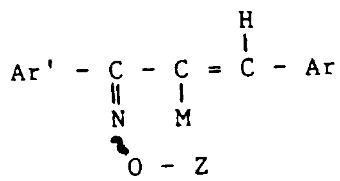

(Ia)

in which Ar, Ar' are as defined hereinbefore and Z represents hydrogen or a $-(CH_2)_n-X$ group is then either:

- when Z is hydrogen and in the presence of a basic condensation agent, treated with an amine having the formula:

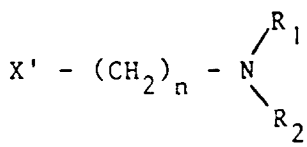

(V)

in which $R_1$ and $R_2$ are as defined hereinbefore and X' is a starting group, or

- when Z represents a group $-(CH_2)_n-X$ where X is as defined hereinbefore, treated with an amine having the formula:

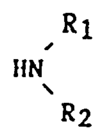

where $R_1$ and $R_2$ are as defined hereinbefore, and

c) the product obtained as in (a) or (b) is converted if required into one of its salts.

**12.** Method for preparing pharmaceutical compositions, characterized in that it consists in adding an efficient amount of a compound according to any one of claims 1 to 10, to a suitable excipient.

**13.** Method according to claim 12 for preparing pharmaceutical compositions in the form of a dose unit.

**14.** Method according to one of claims 12 or 13, for preparing pharmaceutical compositions containing 0.1 to 500 mg of active principle per unit dose.

**15.** Method according to any one of claims 12 to 14, for preparing pharmaceutical compositions containing 2.5 to 125 mg of active principle per unit dose.

**16.** Intermediates useful in preparing propenone oxime ethers according to claim 1 and having one of the following formulae:

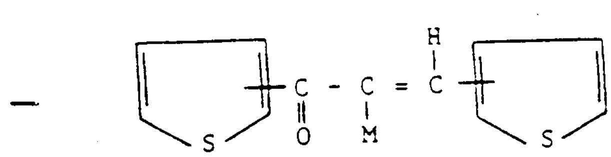

in which M represents a hydrogen atom or a chlorine or bromine atom, or a straight or branched lower alkyl group containing 1 to 6 carbon atoms;

— 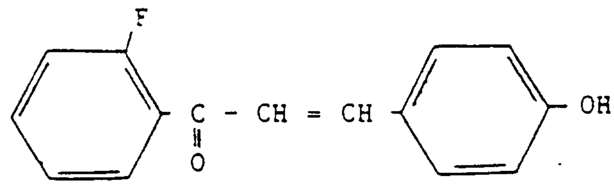

— 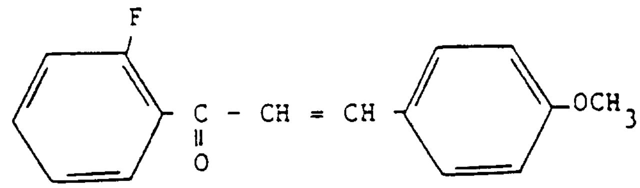

— 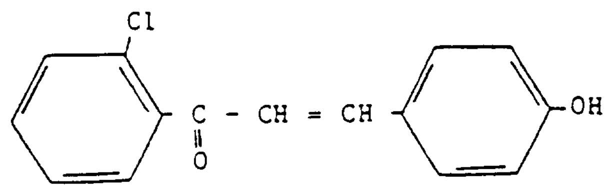 .

**Claims for the following Contracting State : ES**

1. Method for preparing propenone ethers having the trans geometry with respect to the double ethylene bond and having the formula:

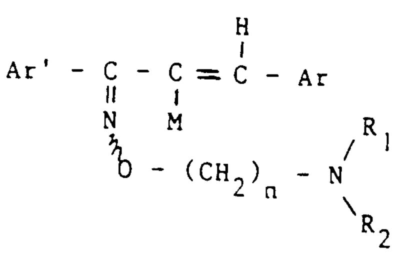

(I)

in which

- Ar and Ar' can each independently denote either:
  (a) a phenyl group, non-substituted or mono or polysubstituted by a halogen atom, a lower alkyl grouping (containing 1 to 4 carbon atoms), a nitro, hydroxyl, alkoxy (1 to 4 carbon atoms), acyloxy (1 to 4 carbon atoms), dimethylamino grouping or a carboxyalkoxy grouping in which the alkylene contains 1 to 4 carbon atoms; or a 9-anthryl group or a naphthyl group, or
  (b) a heteroaromatic group chosen from the pyridyl, thienyl or furyl groups;
- $R_1$ and $R_2$ each independently denote a hydrogen atom or a lower alkyl grouping (1 to 4 carbon atoms) or
  $R_1$ and $R_2$ together with the nitrogen atom to which they are bonded constitute a 1-pyrrolidinyl or piperidino or morpholino or 1-piperazinyl grouping;
- M represents a hydrogen atom or a chlorine or bromine atom, or a straight or branched lower alkyl group containing 1 to 6 carbon atoms;
- n = 2 or 3;

and their salts with mineral or organic acids, characterized in that:

a) a chalcone having the formula:

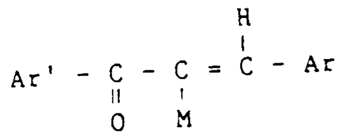

in which Ar and Ar' are as defined hereinabove, is treated with a hydroxylamine having the formula:

$H_2NOZ$ (III)

in which Z represents either

- an aminoalkyl chain having the formula:

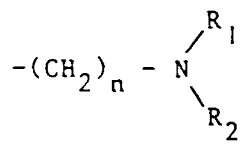

  where $R_1$ and $R_2$ are as defined hereinabove, or
- a hydrogen atom or
- a substituted alkyl group having the formula $-(CH_2)_nX$, where n is as defined hereinabove, and where X represents a starting group;

b) the resulting product having the formula:

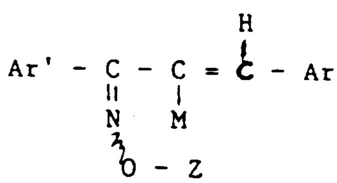

(Ia)

in which Ar, Ar' are as defined hereinbefore and Z represents hydrogen or a $-(CH_2)_n-X$ group is then either:

- when Z is hydrogen and in the presence of a basic condensation agent, treated with an amine having the formula:

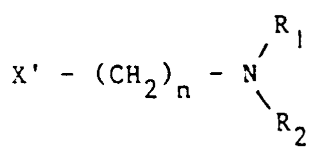

(V)

  in which $R_1$ and $R_2$ are as defined hereinbefore and X' is a starting group, or
- when Z represents a group $-(CH_2)_n-X$ where X is as defined hereinbefore, treated with an amine having the formula:

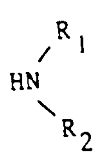

where $R_1$ and $R_2$ are as defined hereinbefore, and

c) the product obtained in (a) or (b) is converted if required into one of its salts with mineral or organic acids.

2. Method of preparation according to claim 1, characterized in that in step a) a chalcone is treated having the formula:

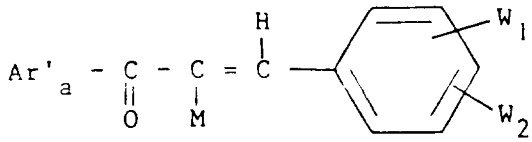

in which $Ar'_a$ represents a group chosen from pyridyl, thienyl, furyl or 9-anthryl and $W_1$ and $W_2$ each independently represent a hydrogen atom or a halogen atom or a lower alkyl grouping (1 to 4 carbon atoms) or a nitro or hydroxyl or alkoxy (1 to 4 carbon atoms) or acyloxy (1 to 4 carbon atoms) or dimethylamino grouping or a carboxyalkoxy grouping in which the alkylene contains 1 to 4 carbon atoms; M being as defined in claim 1.

3. Method of preparation according to claim 1, characterized in that in step a) a chalcone is treated having the formula:

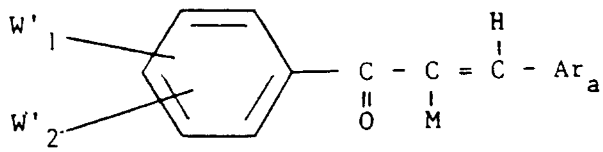

in which $Ar_a$ represents a group chosen from pyridyl, thienyl, furyl or 9-anthryl and $W'_1$ and $W'_2$ each independently represent a hydrogen atom or a halogen atom or a lower alkyl grouping (1 to 4 carbon atoms) or a nitro, hydroxyl, alkoxy (1 to 4 carbon atoms), acyloxy (1 to 4 carbon atoms), dimethylamino grouping or a carboxyalkoxy grouping in which the alkylene contains 1 to 4 carbon atoms; M being as defined in claim 1.

4. Method of preparation according to claim 1, characterized in that in step a) a chalcone is treated having the formula:

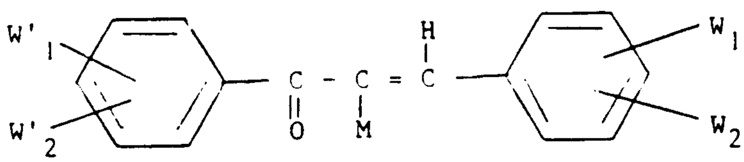

in which $W_1$, $W_2$, $W'_1$, $W'_2$ can each independently denote a hydrogen atom, or a halogen atom or a lower alkyl grouping (1 to 4 carbon atoms) or a nitro or hydroxyl or alkoxy (1 to 4 carbon atoms) or acyloxy (1 to 4 carbon atoms) or dimethylamino grouping or a carboxyalkoxy grouping in which the alkylene contains 1 to 4 carbon atoms, and M is as defined in claim 1.

5. Method of preparation according to claim 1, characterized in that in step a) a chalcone is treated having the formula:

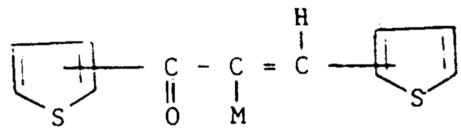

in which the substituents are a 2-thienyl or a 3-thienyl, and M is as defined in claim 1.

**6.** Method of preparation according to claim 1, characterized in that

a) a chalcone having the formula:

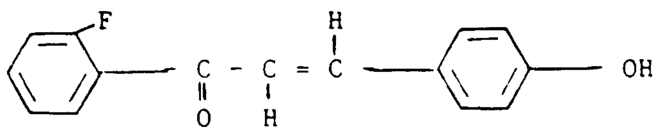

is treated with a hydroxylamine having the formula:

$H_2NOZ$ (III)

in which Z represents either

- an aminoalkyl chain having the formula:

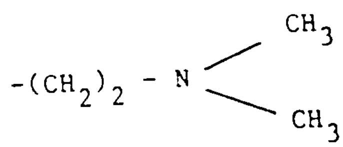

- a hydrogen atom or
- a substituted alkyl group having the formula:
- $(CH_2)_2X$ where X represents a starting group;

b) the resulting product having the formula:

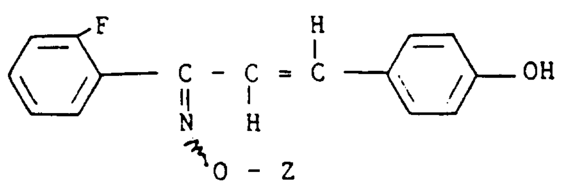

(1a)

in which Z represents hydrogen or a $-(CH_2)_2X$ group is then either:

- when Z is hydrogen treated with an amine having the formula:

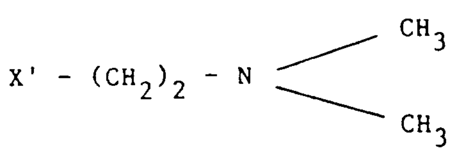

in which X' is a starting group, or

- when Z represents a group $(CH_2)_2X$ treated with an amine having the formula:

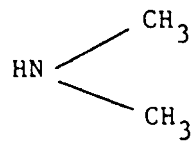

and c) the product obtained as in (a) or (b) having the formula:

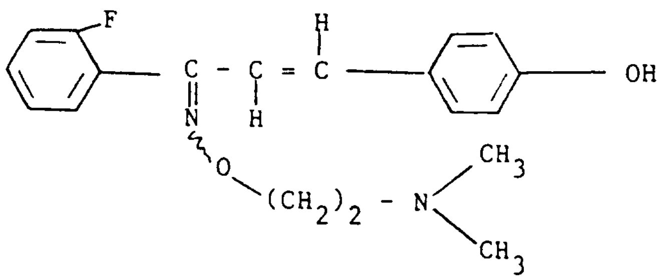

(1)

is converted if required into one of its salts such as methane sulphonate or hemifumarate.

**7.** Method of preparation according to claim 1, characterized in that
a) the chalcone having the formula:

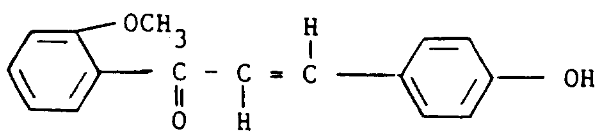

is treated with a hydroxylamine having the formula:

$H_2NOZ$ (III)

in which Z is as defined in claim 6,
b) the resulting product having the formula:

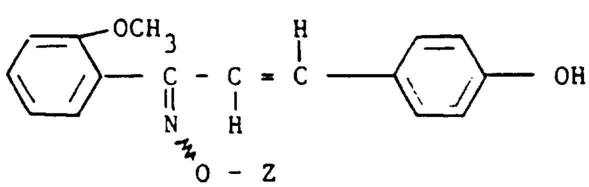

(1a)

in which Z represents hydrogen or a $-(CH_2)_2X$ group is then treated as indicated in claim 6b);

c) the product obtained in a) or b) having the formula:

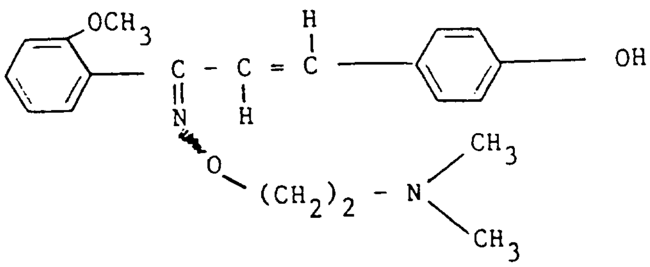 (1)

is converted if required into one of its salts.

**8.** Method of preparation according to claim 1, characterized in that
a) a chalcone having the formula:

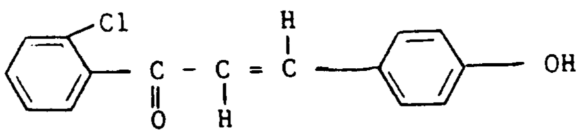

is treated with a hydroxylamine having the formula: $H_2NOZ$ (III) in which Z is as defined in claim 6,
b) the resulting product having the formula:

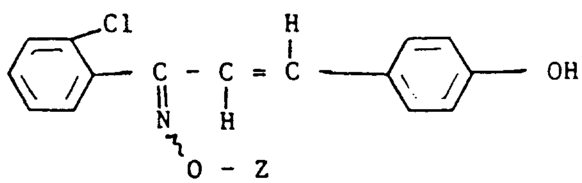

in which Z represents hydrogen or a $-(CH_2)_2X$ group is then treated as indicated in claim 6b),
c) the product obtained in a) or b) having the formula:

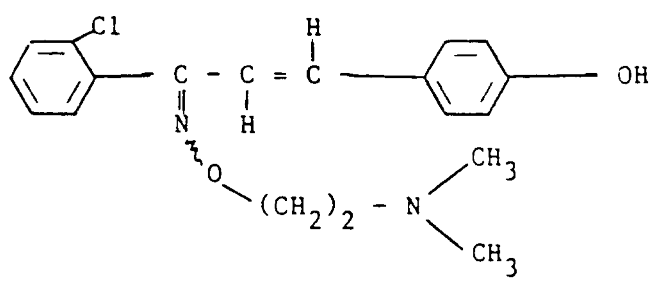 (1)

is converted if required into one of its salts.

**9.** Method for preparing intermediates useful in preparing according to claim 1 propenone oxime ethers of formula (I) and having one of the following formulae:

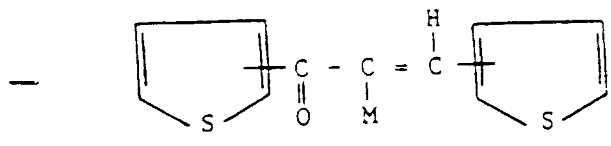

in which M represents a hydrogen atom or a chlorine or bromine atom, or a straight or branched lower alkyl group containing 1 to 6 carbon atoms;

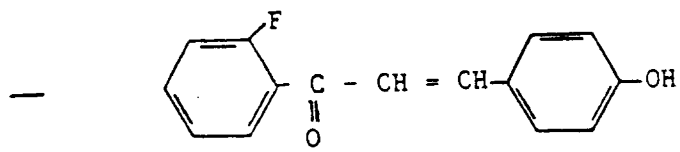

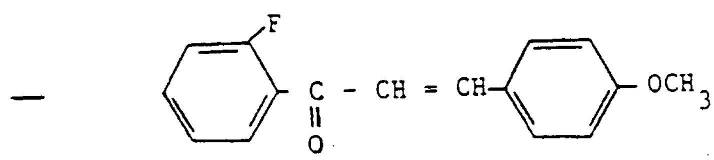

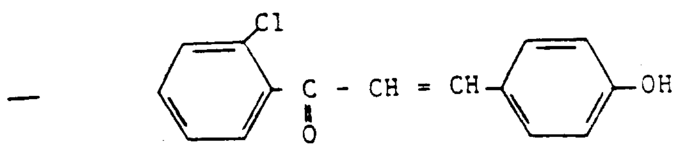

of the type:

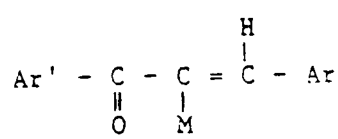

characterized in that it consists in condensing the corresponding cetone of formula Ar'-CO-Alk in which Alk is an alkyl group having 1 to 7 carbon atoms with the corresponding aldehyde of formula Ar-CHO.

**10.** Method for preparing pharmaceutical compositions, characterized in that it consists in adding an efficient amount of a compound of formula (I) or of one of its pharmaceutically acceptable salts, prepared according to any one of claims 1 to 8, to a suitable excipient.

**11.** Method according to claim 10 for preparing pharmaceutical compositions in the form of a dose unit.

**12.** Method according to one of claims 10 or 11 for preparing pharmaceutical compositions containing from 0.1 to 500 mg of active principle per dose unit.

**13.** Method according to any one of claims 10 to 12 for preparing pharmaceutical compositions containing from 2.5 to 125 mg of active principle per dose unit.

**Patentansprüche**

**Patentansprüche für folgende Vertragsstaaten : AT, BE, CH, DE, FR, GB, IT, LI, LU, NL, SE**

**1.** Propenonoximether mit trans-Geometrie in bezug auf die ethylenische Doppelbindung der Formel

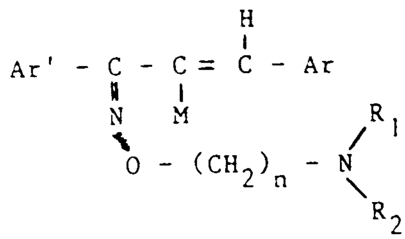

(I),

worin

- Ar und Ar' jeweils unabhängig folgende Bedeutungen haben können:
  a) eine unsubstituierte oder durch ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy-(mit 1 bis 4 Kohlenstoffatomen), Dimethylamino- und Carboxyalkoxygruppe, wobei die Alkylengruppe 1 bis 4 Kohlenstoffatome enthält, mono- oder polysubstituierte Phenylgruppe; oder die 9-Anthryl- oder eine Naphthylgruppe;
  b) eine heteroaromatische Gruppe ausgewählt aus den Gruppen Pyridyl, Thienyl oder Furyl;
- $R_1$ und $R_2$ jeweils unabhängig ein Wasserstoffatom oder eine nied.Alkylgruppe (mit 1 bis 4 Kohlenstoffatomen) darstellen oder $R_1$ und $R_2$ zusammmen mit dem Stickstoffatom, an das sie gebunden sind, eine 1-Pyrrolidinyl-, Piperidino-, Morpholino- oder 1-Piperazinylgruppe bilden;
- M ein Wasserstoffatom, ein Chlor- oder Bromatom oder eine gerad- oder verzweigtkettige nied.Alkylgruppe mit 1 bis 6 Kohlenstoffatomen bedeutet und
- n 2 oder 3 ist,

sowie deren Salze mit Mineral- oder organischen Säuren.

**2.** Propenonoximether nach Anspruch 1 der Formel

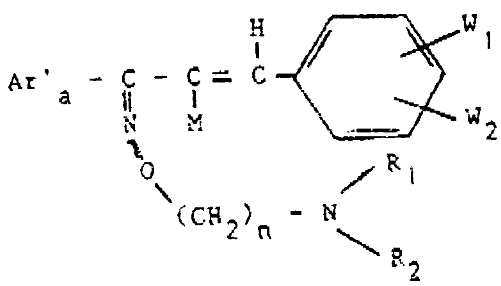

(Ib),

worin Ar'$_a$ eine Gruppe ausgewählt aus Pyridyl, Thienyl, Furyl oder 9-Anthryl bedeutet und $W_1$ und $W_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethylamino- oder Carboxyalkoxygruppe, wobei die Alkylengruppe 1 bis 4 Kohlenstoffatome enthält, darstellen, oder eines seiner Salze mit Mineral- oder organischen Säuren.

**3.** Propenonoximether nach Anspruch 1 der Formel

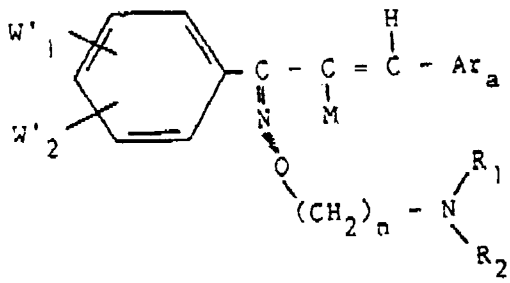

(Ic),

worin $Ar_a$ eine Gruppe ausgewählt aus Pyridyl, Thienyl, Furyl oder 9-Anthryl bedeutet und $W'_1$ und $W'_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethylamino- oder Carboxyalkoxygruppe, wobei die Alkylengruppe 1 bis 4 Kohlenstoffatome enthält, darstellen, oder eines seiner Salze mit Mineral- oder organischen Säuren.

**4.** Propenonoximether nach Anspruch 1 der Formel

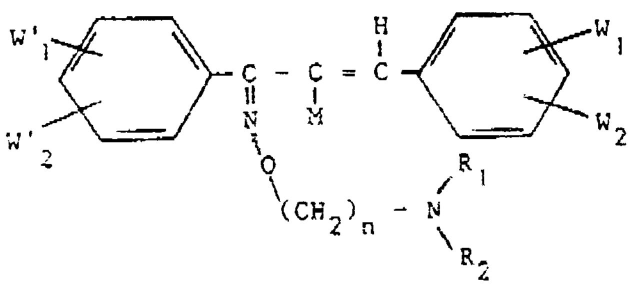

(Id),

worin $W_1$, $W_2$, $W'_1$ und $W'_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethyiamino- oder Carboxyalkoxygruppe, wobei die Alkylengruppe 1 bis 4 Kohlenstoffatome enthält, darstellen, oder eines seiner Salze mit Mineral- oder organischen Säuren.

**5.** Propenonoximether nach Anspruch 1 der Formel

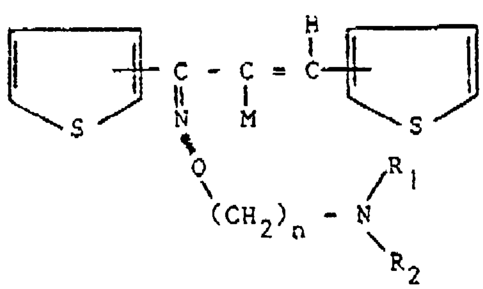

(Ie),

worin die Substituenten 2-Thienyl- oder 3-Thienyl sind, oder eines seiner Salze mit Mineral- oder organischen Säuren.

**6.** syn-trans-N,N-1-Dimethylaminoethoxyimino-1-(2-fluorphenyl)-3-(4-hydroxyphenyl)-2-propen und dessen Salze mit Mineral- oder organischen Säuren.

**7.** syn-trans-N,N-1-Dimethylaminoethoxyimino-1-(2-fluorphenyl)-3-(4-hydroxyphenyl)-2-propen-methansulfonat.

**8.** syn-trans-N,N-1-Dimethylaminoethoxyimino-1-(2-fluorphenyl)-3-(4-hydroxyphenyl)-2-propen-hemifumarat.

**9.** syn-trans-N,N-1-Dimethylaminoethoxyimino-1-(2-methoxyphenyl)-3-(4-hydroxyphenyl)-2-propen und dessen Salze mit Mineral- oder organischen Säuren.

**10.** syn-trans-N,N-1-Dimethylaminoethoxyimino-1-(2-chlorphenyl)-3-(4-hydroxyphenyl)-2-propen und dessen Salze mit Mineral- oder organischen Säuren.

**11.** Verfahren zur Herstellung der Verbindungen nach Anspruch 1, dadurch gekennzeichnet, daß man

a) ein Chalkon der Formel

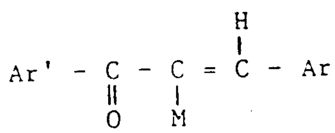

,

worin Ar und Ar' wie in Anspruch 1 definiert sind, mit einem Hydroxylamin der Formel

$H_2NOZ$ (III),

worin Z

- eine Aminoalkylkette der Formel

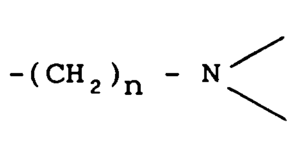

,

  wobei $R_1$ und $R_2$ wie in Anspruch 1 definiert sind,
- ein Wasserstoffatom,
- eine substituierte Alkylgruppe der Formel $-(CH_2)_nX$, wobei n wie in Anspruch 1 definiert ist und X eine Abgangsgruppe darstellt, bedeutet,

behandelt,

b) danach das so erhaltene Produkt der Formel

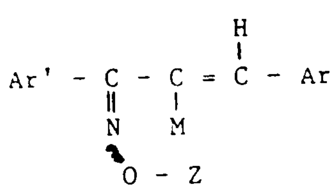

(Ia),

worin Ar und Ar' wie oben definiert sind und Z Wasserstoff oder eine Gruppe $-(CH_2)_nX$ darstellt,

- entweder, wenn Z Wasserstoff ist, in Anwesenheit eines basischen Kondensationsmittels mit einem Amin der Formel

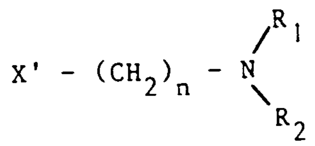

(V),

worin $R_1$ und $R_2$ wie oben definiert sind und X' eine Abgangsgruppe darstellt;

- oder, wenn Z eine Gruppe $-(CH_2)_n-X$ ist, wobei X wie oben definiert ist, mit einem Amin der Formel

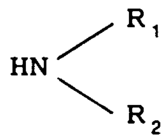

,

worin $R_1$ und $R_2$ wie oben definiert sind, behandelt und

c) gegebenenfalls das in a) oder b) erhaltene Produkt in eines seiner Salze überführt.

12. Pharmazeutische Zusammensetzung, die als Wirkstoff eine Verbindung nach einem der Ansprüche 1 bis 10 enthält.

13. Pharmazeutische Zusammensetzung nach Anspruch 12 in Form einer Dosierungseinheit, in der der Wirkstoff mit einem pharmazeutischen Exzipienten gemischt ist.

14. Pharmazeutische Zusammensetzung nach einem der Ansprüche 12 oder 13, die 0,1 bis 500 mg Wirkstoff pro Dosierungseinheit enthält.

15. Pharmazeutische Zusammensetzung nach einem der Ansprüche 12 bis 14, die 2,5 bis 125 mg Wirkstoff pro Dosierungseinheit enthält.

16. Zwischenprodukte, die bei der Herstellung der Propenonoximether nach Anspruch 1 verwendbar sind, entsprechend einer der nachstehenden Formeln

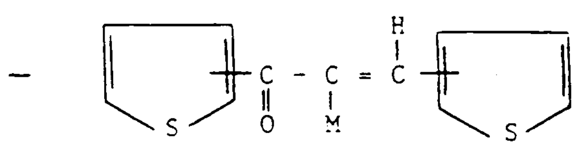

,

worin M ein Wasserstoffatom, ein Chlor- oder Bromatom oder eine gerad- oder verzweigtkettige nied.Alkylgruppe mit 1 bis 6 Kohlenstoffatomen darstellt;

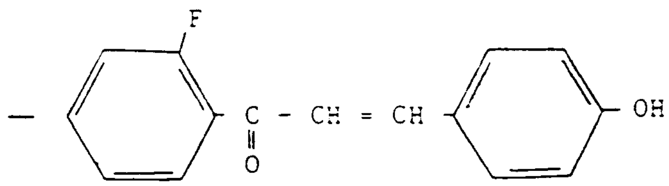

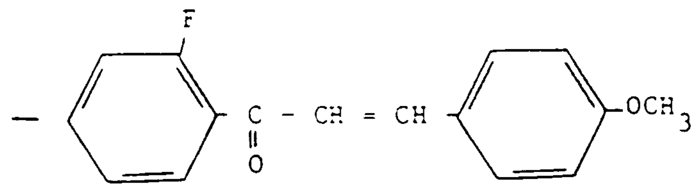

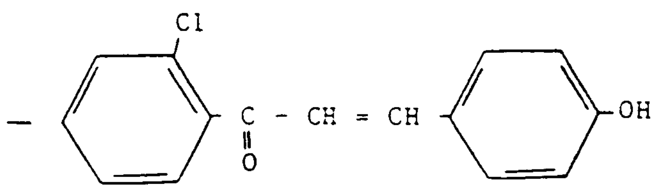

**Patentansprüche für folgenden Vertragsstaat : GR**

1. Propenonoximether mit trans-Geometrie in bezug auf die ethylenische Doppelbindung der Formel

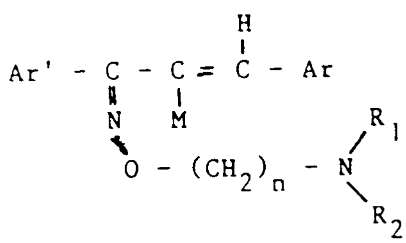

(I),

worin

- Ar und Ar' jeweils unabhängig folgende Bedeutungen haben können:
  a) eine unsubstituierte oder durch ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy-(mit 1 bis 4 Kohlenstoffatomen), Dimethylamino- und Carboxyalkoxygruppe, wobei die Alkylengruppe 1 bis 4 Kohlenstoffatome enthalt, mono- oder polysubstituierte Phenylgruppe; oder die 9-Anthryl- oder eine Naphthylgruppe;
  b) eine heteroaromatische Gruppe ausgewählt aus den Gruppen Pyridyl, Thienyl oder Furyl;
- $R_1$ und $R_2$ jeweils unabhängig ein Wasserstoffatom oder eine nied.Alkylgruppe (mit 1 bis 4 Kohlenstoffatomen) darstellen oder $R_1$ und $R_2$ zusammmen mit dem Stickstoffatom, an das sie gebunden sind, eine 1-Pyrrolidinyl-, Piperidino-, Morpholino- oder 1-Piperazinylgruppe bilden;
- M ein Wasserstoffatom, ein Chlor- oder Bromatom oder eine gerad- oder verzweigtkettige nied.Alkylgruppe mit 1 bis 6 Kohlenstoffatomen bedeutet und
- n 2 oder 3 ist,

sowie deren Salze mit Mineral- oder organischen Säuren.

**2.** Propenonoximether nach Anspruch 1 der Formel

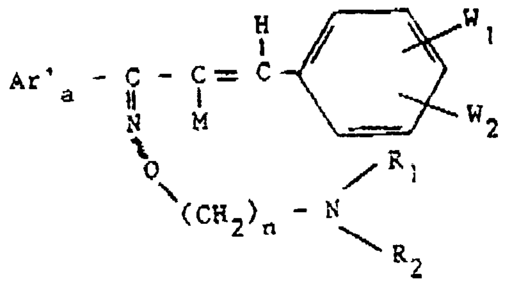

(Ib),

worin $Ar'_a$ eine Gruppe ausgewählt aus Pyridyl, Thienyl, Furyl oder 9-Anthryl bedeutet und $W_1$ und $W_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethylamino- oder Carboxyalkoxygruppe, wobei die Alkylengruppe 1 bis 4 Kohlenstoffatome enthält, darstellen, oder eines seiner Salze mit Mineral- oder organischen Säuren.

**3.** Propenonoximether nach Anspruch 1 der Formel

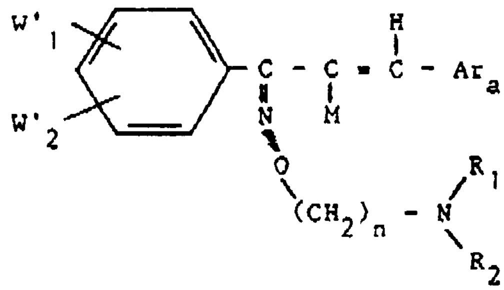

(Ic),

worin $Ar_a$ eine Gruppe ausgewählt aus Pyridyl, Thienyl, Furyl oder 9-Anthryl bedeutet und $W'_1$ und $W'_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethylamino- oder Carboxyalkoxygruppe, wobei die Alkylengruppe 1 bis 4 Kohlenstoffatome enthält, darstellen, oder eines seiner Salze mit Mineral- oder organischen Säuren.

**4.** Propenonoximether nach Anspruch 1 der Formel

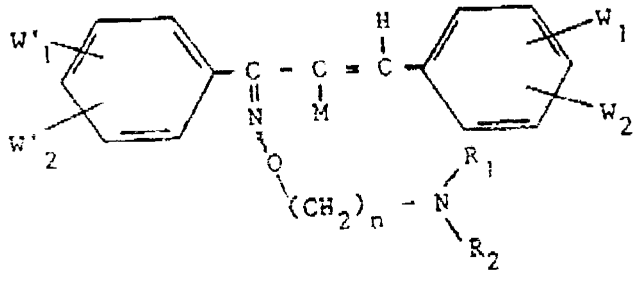

(Id),

worin $W_1$, $W_2$, $W'_1$ und $W'_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethylamino- oder Carboxyalkoxygruppe, wobei die Alkylengruppe 1 bis 4 Kohlenstoffatome enthält, darstellen, oder eines seiner Salze mit Mineral- oder organischen Säuren.

**5.** Propenonoximether nach Anspruch 1 der Formel

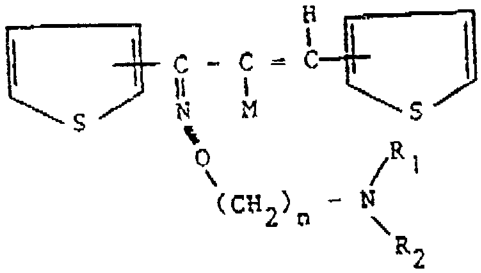

(Ie),

worin die Substituenten 2-Thienyl- oder 3-Thienyl sind, oder eines seiner Salze mit Mineral- oder organischen Säuren.

**6.** syn-trans-N,N-1-Dimethylaminoethoxyimino-1-(2-fluorphenyl)-3-(4-hydroxyphenyl)-2-propen und dessen Salze mit Mineral- oder organischen Säuren.

**7.** syn-trans-N,N-1-Dimethylaminoethoxyimino-1-(2-fluorphenyl)-3-(4-hydroxyphenyl)-2-propen-methansulfonat.

**8.** syn-trans-N,N-1-Dimethylaminoethoxyimino-1-(2-fluorphenyl)-3-(4-hydroxyphenyl)-2-propen-hemifumarat.

**9.** syn-trans-N,N-1-Dimethylaminoethoxyimino-1-(2-methoxyphenyl)-3-(4-hydroxyphenyl)-2-propen und dessen Salze mit Mineral- oder organischen Säuren.

**10.** syn-trans-N,N-1-Dimethylaminoethoxyimino-1-(2-chlorphenyl)-3-(4-hydroxyphenyl)-2-propen und dessen Salze mit Mineral- oder organischen Säuren.

**11.** Verfahren zur Herstellung der Verbindungen nach Anspruch 1, dadurch gekennzeichnet, daß man
a) ein Chalkon der Formel

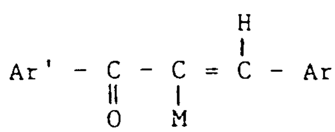

,

worin Ar und Ar' wie in Anspruch 1 definiert sind, mit einem Hydroxylamin der Formel

$H_2NOZ$ (III),

worin Z
- eine Aminoalkylkette der Formel

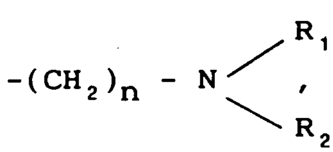

,

wobei $R_1$ und $R_2$ wie in Anspruch 1 definiert sind,
- ein Wasserstoffatom,
- eine substituierte Alkylgruppe der Formel $-(CH_2)_nX$, wobei n wie in Anspruch 1 definiert ist und X eine Abgangsgruppe darstellt, bedeutet,

behandelt,
b) danach das so erhaltene Produkt der Formel

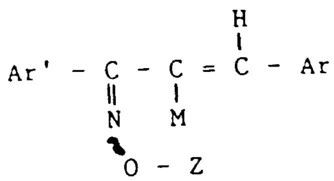

(Ia),

worin Ar und Ar' wie oben definiert sind und Z Wasserstoff oder eine Gruppe $-(CH_2)_nX$ darstellt,

- entweder, wenn Z Wasserstoff ist, in Anwesenheit eines basischen Kondensationsmittels mit einem Amin der Formel

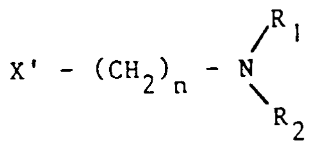

(V),

worin $R_1$ und $R_2$ wie oben definiert sind und X' eine Abgangsgruppe darstellt;

- oder, wenn Z eine Gruppe $-(CH_2)_n-X$ ist, wobei X wie oben definiert ist, mit einem Amin der Formel

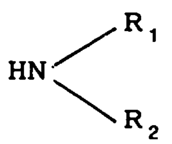

,

worin $R_1$ und $R_2$ wie oben definiert sind, behandelt und

c) gegebenenfalls das in a) oder b) erhaltene Produkt in eines seiner Salze überführt.

**12.** Verfahren zur Herstellung von pharmazeutischen Zusammensetzungen, dadurch gekennzeichnet, daß man eine wirksame Menge einer Verbindung nach einem der Ansprüche 1 bis 10 in einen geeigneten Exzipienten einarbeitet.

**13.** Verfahren nach Anspruch 12 zur Herstellung von pharmazeutischen Zusammensetzungen in Form einer Dosierungseinheit.

**14.** Verfahren nach einem der Ansprüche 12 oder 13 zur Herstellung von pharmazeutischen Zusammensetzungen, die 0,1 bis 500 mg Wirkstoff pro Dosierungseinheit enthalten.

**15.** Verfahren nach den Ansprüchen 12 bis 14 zur Herstellung von pharmazeutischen Zusammensetzungen, die 2,5 bis 125 mg Wirkstoff pro Dosierungseinheit enthalten.

**16.** Zwischenprodukte, die bei der Herstellung der Propenonoximether nach Anspruch 1 verwendbar sind, entsprechend einer der nachstehenden Formeln

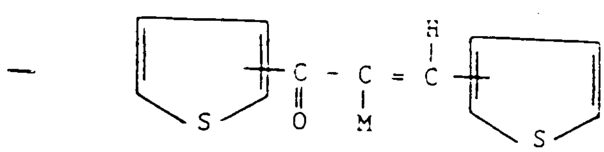

,

worin M ein Wasserstoffatom, ein Chlor- oder Bromatom oder eine gerad- oder verzweigtkettige nied.Alkylgruppe mit 1 bis 6 Kohlenstoffatomen darstellt;

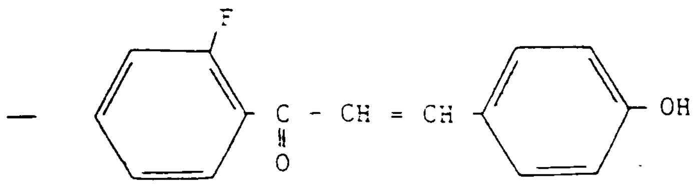

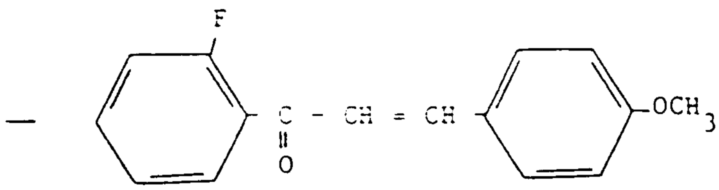

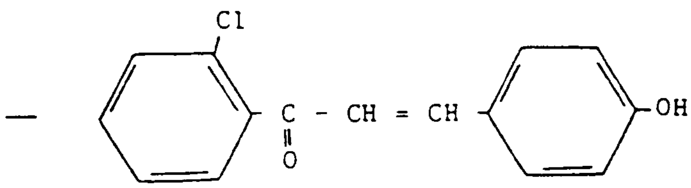

## Patentansprüche für folgende Vertragsstaat : ES

**1.** Verfahren zur Herstellung von Propenonoximethern mit trans-Geometrie in bezug auf die ethylenische Doppelbindung der Formel

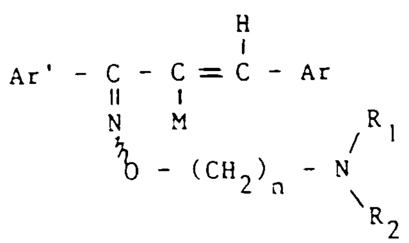

(I),

worin

- Ar und Ar' jeweils unabhängig folgende Bedeutungen haben können:
  a) eine unsubstituierte oder durch ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy-(mit 1 bis 4 Kohlenstoffatomen), Dimethylamino- und Carboxyalkoxygruppe, wobei die Alkylengruppe 1 bis 4 Kohlenstoffatome enthält, mono- oder polysubstituierte Phenylgruppe; oder die 9-Anthryl- oder eine Naphthylgruppe;
  b) eine heteroaromatische Gruppe ausgewählt aus den Gruppen Pyridyl, Thienyl oder Furyl;
- $R_1$ und $R_2$ jeweils unabhängig ein Wasserstoffatom oder eine nied.Alkylgruppe (mit 1 bis 4 Kohlenstoffatomen) darstellen oder $R_1$ und $R_2$ zusammmen mit dem Stickstoffatom, an das sie gebunden sind, eine 1-Pyrrolidinyl-, Piperidino-, Morpholino- oder 1-Piperazinylgruppe bilden;
- M ein Wasserstoffatom, ein Chlor- oder Bromatom oder eine gerad- oder verzweigtkettige nied.Alkylgruppe mit 1 bis 6 Kohlenstoffatomen bedeutet und
- n 2 oder 3 ist,

sowie deren Salzen mit Mineral- oder organischen Säuren, dadurch gekennzeichnet, daß man

a) ein Chalkon der Formel

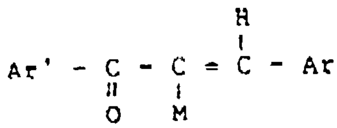

,

worin Ar und Ar' wie oben definiert sind, mit einem Hydroxylamin der Formel

$H_2NOZ$ (III),

worin Z

- eine Aminoalkylkette der Formel

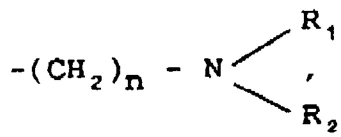

wobei $R_1$ und $R_2$ wie oben definiert sind,
- ein Wasserstoffatom,
- eine substituierte Alkylgruppe der Formel $-(CH_2)_nX$, wobei n wie oben definiert ist und X eine Abgangsgruppe darstellt, bedeutet,

behandelt,

b) danach das so erhaltene Produkt der Formel

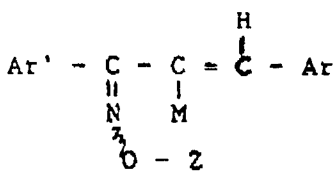

(Ia),

worin Ar und Ar' wie oben definiert sind und Z Wasserstoff oder eine Gruppe $-(CH_2)_n-X$ darstellt,

- entweder, wenn Z Wasserstoff ist, in Anwesenheit eines basischen Kondensationsmittels mit einem Amin der Formel

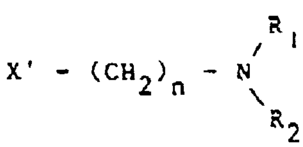

(V),

worin $R_1$ und $R_2$ wie oben definiert sind und X' eine Abgangsgruppe darstellt;
- oder, wenn Z eine Gruppe $-(CH_2)_n-X$ ist, wobei X wie oben definiert ist, mit einem Amin der Formel

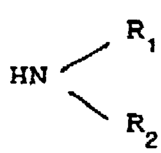

worin $R_1$ und $R_2$ wie oben definiert sind, behandelt und

c) gegebenenfalls das in a) oder b) erhaltene Produkt in eines seiner Salze mit Mineral- oder organischen Säuren überführt.

2. Herstellungsverfahren nach Anspruch 1, dadurch gekennzeichnet, daß man in Schritt a) ein Chalkon der Formel

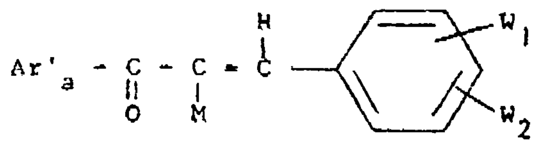

Worin Ar'$_a$ eine Gruppe ausgewählt aus Pyridyl, Thienyl, Furyl oder 9-Anthryl bedeutet und $W_1$ und $W_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethylamino- oder Carboxyalkoxygruppe, wobei die Alkylengruppe 1 bis 4 Kohlenstoffatome enthält, darstellen und M wie in Anspruch 1 definiert ist, behandelt.

3. Herstellungsverfahren nach Anspruch 1, dadurch gekennzeichnet, daß man in Schritt a) ein Chalkon der Formel

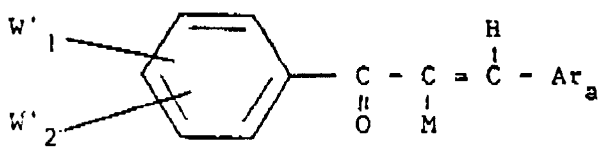

worin $Ar_a$ eine Gruppe ausgewählt aus Pyridyl, Thienyl, Furyl oder 9-Anthryl bedeutet und $W'_1$ und $W'_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethylamino- oder Carboxyalkoxygruppe, wobei die Alkylengruppe 1, bis 4 Kohlenstoffatome enthält, darstellen und M wie in Anspruch 1 definiert ist, behandelt.

4. Herstellungsverfahren nach Anspruch 1, dadurch gekennzeichnet, daß man in Schritt a) ein Chalkon der Formel

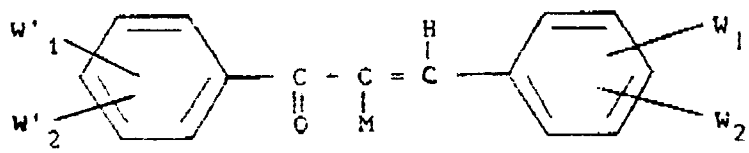

worin $W_1$, $W_2$, $W'_1$ und $W'_2$ jeweils unabhängig ein Wasserstoffatom, ein Halogenatom, eine nied.Alkylgruppe (mit 1 bis 4 Kohlenstoffatomen), eine Nitro-, Hydroxyl-, Alkoxy- (mit 1 bis 4 Kohlenstoffatomen), Acyloxy- (mit 1 bis 4 Kohlenstoffatomen), Dimethylamino- oder Carboxyalkoxygruppe, wobei die Alkylengruppe 1 bis 4 Kohlenstoffatome enthält, darstellen und M wie in Anspruch 1 definiert ist, behandelt.

5. Herstellungsverfahren nach Anspruch 1, dadurch gekennzeichnet, daß man in Schritt a) ein Chalkon der Formel

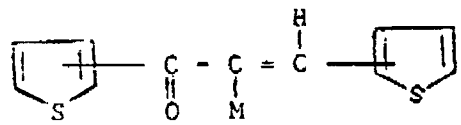
,

worin die Substituenten 2-Thienyl- oder 3-Thienyl sind und M wie in Anspruch 1 definiert ist, behandelt.

**6.** Herstellungsverfahren nach Anspruch 1, dadurch gekennzeichnet, daß man

a) ein Chalkon der Formel

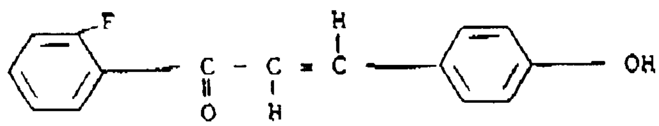
,

mit einem Hydroxylamin der Formel

$H_2NOZ$ (III),

worin Z

- eine Aminoalkylkette der Formel

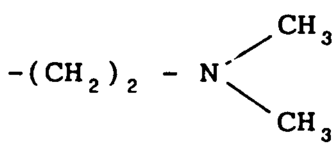
,

- ein Wasserstoffatom,
- eine substituierte Alkylgruppe der Formel $-(CH_2)_2X$, wobei X eine Abgangsgruppe darstellt,

bedeutet,

behandelt,

b) danach das so erhaltene Produkt der Formel

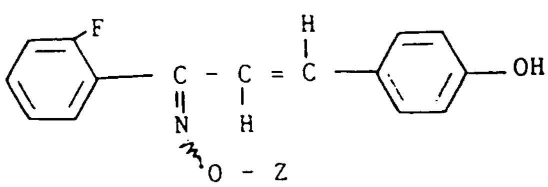
(Ia),

worin Z Wasserstoff oder die Gruppe $-(CH_2)_2X$ darstellt,

- entweder, wenn Z Wasserstoff ist, mit einem Amin der Formel

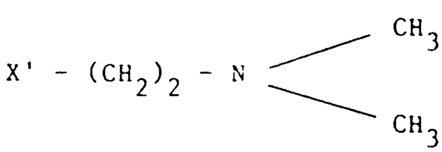
,

worin X' eine Abgangsgruppe darstellt;

- oder, wenn Z eine Gruppe $-(CH_2)_2X$ ist, mit einem Amin der Formel

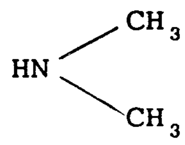

,

behandelt und

c) gegebenenfalls das in a) oder b) erhaltene Produkt der Formel

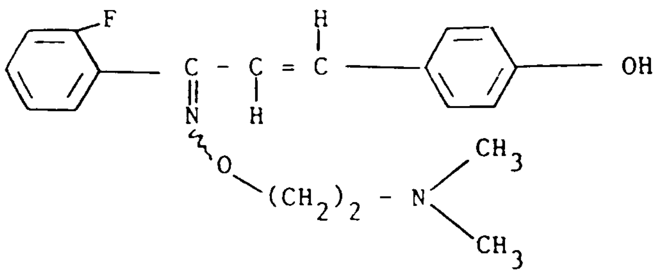

(I)

in eines seiner Salze, wie das Methansulfonat oder Hemifumarat, überführt.

**7.** Herstellungsverfahren nach Anspruch 1, dadurch gekennzeichnet, daß man

a) ein Chalkon der Formel

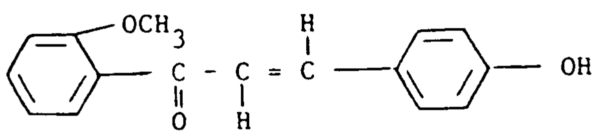

,

mit einem Hydroxylamin der Formel

$H_2NOZ$ (III),

worin Z wie in Anspruch 6 definiert ist, behandelt,

b) danach das so erhaltene Produkt der Formel

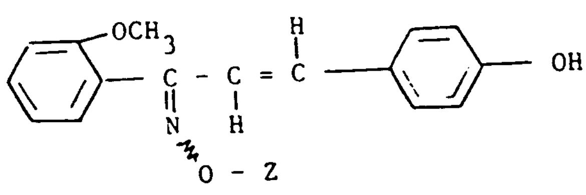

(Ia),

worin Z Wasserstoff oder die Gruppe $-(CH_2)_2X$ darstellt, wie in Anspruch 6b) angegeben, behandelt und

c) gegebenenfalls das in a) oder b) erhaltene Produkt der Formel

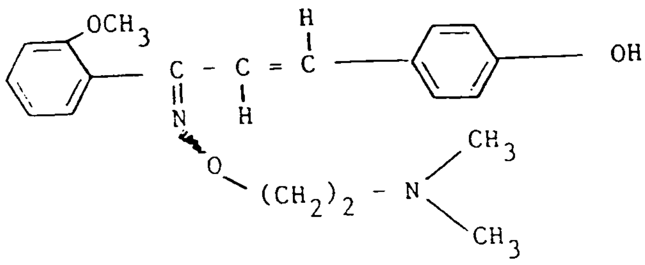

(I)

in eines seiner Salze überführt.

**8.** Herstellungsverfahren nach Anspruch 1, dadurch gekennzeichnet, daß man
a) ein Chalkon der Formel

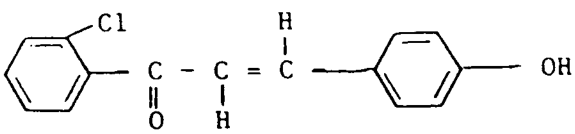

,

mit einem Hydroxylamin der Formel

$H_2NOZ$ (III),

worin Z wie in Anspruch 6 definiert ist, behandelt,
b) danach das so erhaltene Produkt der Formel

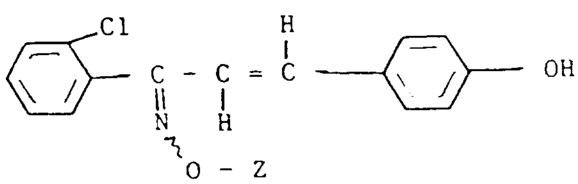

,

worin Z Wasserstoff oder die Gruppe $-(CH_2)_2X$ darstellt, wie in Anspruch 6b) angegeben, behandelt und
c) gegebenenfalls das in a) oder b) erhaltene Produkt der Formel

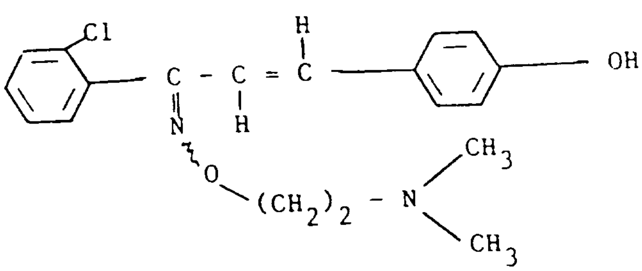

(I)

in eines seiner Salze überführt.

**9.** Verfahren zur Herstellung von Zwischenprodukten, die bei der Herstellung von Propenonoximethern der Formel (I) gemäß Anspruch 1 verwendbar sind und entsprechend einer der nachstehenden Formeln

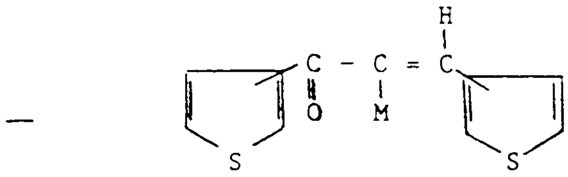,

worin M ein Wasserstoffatom, ein Chlor- oder Bromatom oder eine gerad- oder verzweigtkettige nied.Alkylgruppe mit 1 bis 6 Kohlenstoffatomen bedeutet;

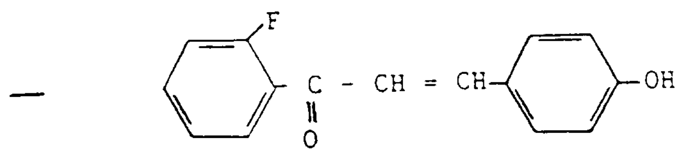

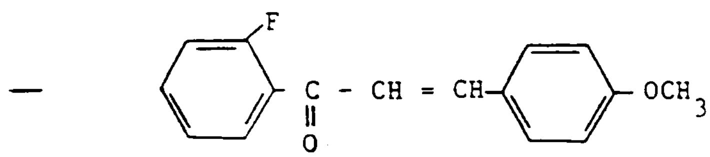

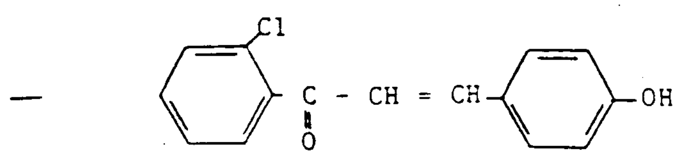

vom Typ

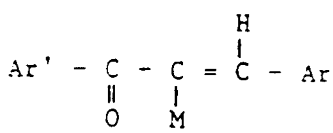,

dadurch gekennzeichnet, daß man das Keton der Formel Ar'-CO-Alk, worin Alk eine Alkylgruppe mit 1 bis 7 Kohlenstoffatomen bedeutet, mit einem Aldehyd der Formel Ar-CHO kondensiert.

**10.** Verfahren zur Herstellung von pharmazeutischen Zusammensetzungen, dadurch gekennzeichnet, daß man eine wirksame Menge einer Verbindung der Formel (I) oder eines ihrer pharmazeutisch annehmbaren Salze, hergestellt nach einem der Ansprüche 1 bis 8, in einen geeigneten Exzipienten einarbeitet.

**11.** Verfahren nach Anspruch 10 zur Herstellung von pharmazeutischen Zusammensetzungen in Form einer Dosierungseinheit.

**12.** Verfahren nach einem der Ansprüche 10 oder 11 zur Herstellung von pharmazeutischen Zusammensetzungen, die 0,1 bis 500 mg Wirkstoff pro Dosierungseinheit enthalten.

**13.** Verfahren nach einem der Ansprüche 10 bis 12 zur Herstellung von pharmazeutischen Zusammensetzungen, die 2,5 bis 125 mg Wirkstoff pro Dosierungseinheit enthalten.